# EUROPEAN PATENT APPLICATION

(11) **EP 3 822 285 A1**
(43) Date of publication of application: **19.05.2021**
(21) Application number: 19208735.1
(22) Date of filing: 12.11.2019
(51) Int. Cl.: C07K 14/315, A23L 3/3526, A61K 38/16

(54) **VARIANT NISIN PEPTIDES**

(71) Applicant: University College Cork-National University of Ireland, Cork, Cork (IE); TEAGASC, The Agriculture and Food Development Authority, Dublin 4 (IE)
(72) Inventor: COTTER, Paul, Fermoy, Co. Cork (IE); SHEEHAN, Diarmuid, Fermoy, Co. Cork (IE); BHAGYA JONNALA, Rekha, Kilworth, Co. Cork (IE); FIELD, Des, Cork (IE); HILL, Colin, Cork (IE); ROSS, Paul, Cork (IE)
(74) Representative: Barker Brettell LLP

(57) **Abstract**

The invention relates to a variant nisin peptide, wherein the variant nisin peptide is a modified variant of wild-type nisin, wherein the modification comprises or consists of an amino acid residue modification selected from: i) M17Q or M17A; ii) T2L; and iii) one set of modified hinge residues selected from a) N20H and M21T, b) N20V, M21G, and K22A, c) N20V, M21G, and K22V or d) N20V, M21G, and K22T; or v) combinations of modifications i), ii), and iii), and related medical uses and products comprising such nisin peptides.

## Description

This invention relates to variant nisin peptides and compositions, and their use in methods of treating or preventing mastitis or other bacterial infection.

Bovine mastitis is a major disease affecting dairy cattle worldwide, and results from an inflammation of the mammary gland. Mastitis is the most common infectious disease among dairy herds and is estimated to cost US dairy farmers US $1.7 billion annually. The causative agents for the inflammatory reaction are microorganisms, with *Staphylococcus aureus* being responsible for 15-30% of the infections.

The use of antibiotics to treat mastitis during lactation is common, as between 2% and 55% of cows encounter a mastitis infection during this period. Notably, the mastitis caused by *S. aureus* is characterized by significantly lower cure rates compared with infections caused by other microorganisms, mainly as a result of unusually frequent acquisition of antibiotic resistance mechanisms by the pathogen. The use of antibiotics to treat animals that are in the food chain can obviously compound this issue by selecting for the development of antibiotic resistance among food microorganisms and by exposing consumers to antibiotic residues in milk and other dairy foods (Doyle et al., 2013). Processing of milk for cheese and yoghurt manufacture is also affected, as bacterial starter cultures are inhibited and the quality of the final product is generally compromised. Furthermore, unpasteurised milk and many dairy products contain a diverse microbiological population. These microorganisms play important roles in dairy foods and can, for example, contribute to the development of flavours and aromas, determine safety, cause spoilage or enhance the health of the consumer.

While the complete elimination of antibiotic therapy for mastitis is unlikely given modern intensive farming practices, a reduction in antibiotic use is highly desirable. Thus new alternatives to conventional antibiotics are much sought after. One group of compounds with considerable potential for preventative and therapeutic use is the lantibiotic class of bacteriocins (bacterially derived antimicrobial peptides). Lantibiotics are ribosomally-synthesized peptides that are characterised by the presence of unusual amino acids including lanthionine and/or methyllanthionine. The most intensively studied lantibiotic is nisin.

Produced by *Lactococcus lactis,* nisin exhibits antibacterial activity against a wide range of Gram-positive bacteria, including foodborne pathogens from the genera *Staphylococcus, Bacillus* and *Clostridium.* Nisin is used as a food preservative in over 50 countries and has been approved in the EU (as additive E234) and by the US Food and Drug Administration (FDA) (Delves-Broughton, 2005). In addition, both nisin A, and its natural variant nisin Z, are effective against the Gram positive pathogens responsible for bovine mastitis and have been incorporated into a number of products (such as Wipe Out® and Mast Out®) dedicated to controlling or treating such infections (Sears *et al.,* 1992, Wu *et al.,* 2007, Cao *et al.,* 2007). Notably, Mast Out®, a Nisin-based treatment for mastitis in lactating cows has been shown to give statistically significant cure rates in an experimental field trial involving 139 cows with subclinical mastitis.

Several examples of bioengineered derivatives of nisin have been generated that provide examples of how nisin functionality can be improved through single, or a small number of residue replacements (Kuipers *et al.,* 1996, Healy *et al.,* 2013, Ge *et al.,* 2016, Li *et al.,* 2018, Zhou *et al.,* 2016). Although nisin has been the subject of mutagenesis studies since the early nineties, more recently, a number of bioengineered nisin derivatives have been generated with academic and potential commercial value that display enhanced activity towards pathogenic bacteria including mastitis-associated S. *aureus* and *S. agalactiae,* as well as many drug-resistant microbes including methicillin-resistant *S*. *aureus* (MRSA), vancomycin-intermediate *S. aureus* (VISA), heterogeneous vancomycin-intermediate *S. aureus* (hVISA) and *Staphylococcus pseudintermedius.* Notably, nisin has been engineered to be resistant to the nisin resistance protein (NSR) which provides resistance through the proteolytic cleavage of nisin and is often associated with streptococci of bovine origin. Importantly, it was also apparent from these studies that several bioengineered nisin derivatives exhibit strain or species-specific enhanced potency. Recognising the therapeutic importance of nisin, there is still need to find further improved nisin variants with improved therapeutic potential.

An aim of the present invention is to provide derivatives of nisin with enhanced properties.

According to a first aspect of the invention, there is provided a variant nisin peptide, wherein the variant nisin peptide is a modified variant of wild-type nisin, wherein the modification comprises or consists of an amino acid residue modification selected from:
i) M17Q or M17A;
ii) T2L; and
iii) one set of modified hinge residues selected from a) N20H and M21T, b) N20V, M21G, and K22A, c) N20V, M21G, and K22V or d) N20V, M21G, and K22T; or
iv) combinations of modifications i), ii) and iii).

The present invention advantageously provides novel derivatives of nisin with distinct functional properties, namely enhanced activity towards pathogenic targets involved in mastitis and reduced activity towards many of the 'friendly' organisms naturally present in milk, i.e. *lactococci* and *lactobacilli.* Bioengineered nisin derivatives are provided that exhibit target specific variations in potency. The use of these peptides as therapeutics for the treatment of mastitis would have many benefits given that milk lactic acid bacteria (LAB) are key to the subsequent generation of fermented dairy products. In addition, in contrast to antibiotics, the presence of nisin residues in milk may not require that the milk be withheld as nisin, being a ribosomal peptide, is susceptible to digestive enzymes and is easily destroyed in the gut. Further advantageously, the novel nisin variants can be used in treating infectious mastitis in lactating mothers. Nisin can effectively reduce staphylococcal numbers in breast milk and lead to the complete disappearance of clinical signs of mastitis after two weeks of treatment. More importantly, nisin has been previously shown to be successful where therapy using traditional antibiotics had failed to provide any improvement in symptoms. Nisin A is already employed commercially as an anti-mastitis product in the form of Wipe Out® and Mast Out® in the veterinary field. Given the potency of the novel nisin variants against mastitis-associated staphylococci, these novel Nisin variant peptides can provide novel anti-mastitis antimicrobials.

The wild-type nisin may comprise or consist of the sequence ITSISLCTPGCKTGALMGCNMKTATCHCSIHVSK (SEQ ID NO: 1). In another embodiment, the wild-type nisin may comprise or consist of the sequence of any of the sequences selected from SEQ ID NO: 21-30.

In one embodiment, the modification may comprise or consist of M17Q and T2L. In one embodiment, the modification may comprise or consist of M17Q, T2L and nisin hinge residues NMK modified to HTK.

In one embodiment, the modifications further comprise a modification to G18, such as G18T or G18S.

According to a second aspect of the invention, there is provided a variant nisin peptide, wherein the variant nisin peptide is a modified variant of wild-type nisin, wherein the modification comprises or consists of an amino acid residue modification selected from:
i) M17Q;
ii) K12T or K12V
iii) N20S
iv) one of M21F, M21A, M21V, M21Y, or M21Q;
v) H27Q; and
vi) one set of modified hinge residues selected from a) N20V, M21G, and K22A, or b) N20P, M21A, and H27Q; or
vii) combinations of modifications i) to vi).

In one embodiment of the first or second aspect, the modifications further comprise a modification to S29, such as S29D or S29E. Additionally or alternatively, the modifications may further comprise a modification to 130, such as I30V.

Advantageously, Nisin peptide variants comprising such modifications have been found to enhance broad antimicrobial activity against *Thermus* target strains. In addition, a number of the variants exhibit enhanced specific activity against a subset of strains. Such peptides can be useful as biopreservatives that can inhibit the growth of *Thermus* for example, preventing the pink discolouration defect caused by the presence of *Thermus.*

In one embodiment, the variant nisin may comprise modifications limited to no more than one modified amino acid residue, two or less modified amino acid residues, three or less modified amino acid residues, four or less modified amino acid residues, five or less modified amino acid residues, six or less modified amino acid residues, ten or less modified amino acid residues, 15 or less modified amino acid residues relative to SEQ ID NO: 1. In another embodiment, the variant nisin may comprise modifications limited to no more than one modified amino acid residue relative to any of SEQ ID NOs: 21-30. In another embodiment, the variant nisin may comprise modifications limited to no more than two modified amino acid residues relative to any of SEQ ID NOs: 21-30. In another embodiment, the variant nisin may comprise modifications limited to three or less modified amino acid residues relative to any of SEQ ID NOs: 21-30. In another embodiment, the variant nisin may comprise modifications limited to four or less modified amino acid residues relative to any of SEQ ID NOs: 21-30.

Amino acid substitutions may be conservative substitutions. For example, a modified residue may comprise substantially similar properties as the wild-type substituted residue. For example, a substituted residue may comprise substantially similar or equal charge or hydrophobicity as the wild-type substituted residue. For example, a substituted residue may comprise substantially similar molecular weight or steric bulk as the wild-type substituted residue.

The wild-type nisin (i.e. prior to modification according to the invention) may comprise Nisin A, wherein Nisin A comprises or consists of a sequence of SEQ ID NO: 1.

In one embodiment the variant nisin molecule may have at least 75% identity with wild-type SEQ ID NO: 1. In another embodiment the variant nisin molecule may have at least 80% identity with wild-type SEQ ID NO: 1. In another embodiment the variant nisin molecule may have at least 85% identity with wild-type SEQ ID NO: 1. In another embodiment the variant nisin molecule may have at least 90% identity with wild-type SEQ ID NO: 1. In another embodiment the variant nisin molecule may have at least 95% identity with wild-type SEQ ID NO: 1. In another embodiment the variant nisin molecule may have at least 98% identity with wild-type SEQ ID NO: 1. In another embodiment the variant nisin molecule may have at least 99% identity with wild-type SEQ ID NO: 1. In another embodiment the variant nisin molecule may have at least 99.5% identity with wild-type SEQ ID NO: 1.

In one embodiment, the amino acid residue modification(s) in the variant nisin molecule, or variants thereof, may be selected from the group consisting of the amino acid at position(s) 2,17 20, 21 and 22 on wild-type Nisin of SEQ ID NO: 1 or corresponding position(s) in other nisin molecules.

In one embodiment, the variant Nisin comprises or consists of any one of the sequences selected from the group comprising:
ITSISLCTPGCKTGALQGCNMKTATCHCSIHVSK (SEQ ID NO: 2);
ILSISLCTPGCKTGALMGCNMKTATCHCSIHVSK (SEQ ID NO: 3);
ITSISLCTPGCKTGALMGCHTKTATCHCSIHVSK (SEQ ID NO: 4);
ITSISLCTPGCKTGALMGCVGATATCHCSIHVSK (SEQ ID NO: 5);
ITSISLCTPGCKTGALMGCVGVTATCHCSIHVSK (SEQ ID NO: 6);
ITSISLCTPGCKTGALMGCVGTTATCHCSIHVSK (SEQ ID NO: 7);
ILSISLCTPGCKTGALQGCNMKTATCHCSIHVSK (SEQ ID NO: 8);
ITSISLCTPGCKTGALQGCHTKTATCHCSIHVSK (SEQ ID NO: 9);
ITSISLCTPGCKTGALQGCVGATATCHCSIHVSK (SEQ ID NO: 10);
ITSISLCTPGCKTGALQGCVGVTATCHCSIHVSK (SEQ ID NO: 11);
ITSISLCTPGCKTGALQGCVGTTATCHCSIHVSK (SEQ ID NO: 12);
ILSISLCTPGCKTGALMGCHTKTATCHCSIHVSK (SEQ ID NO: 13);
ILSISLCTPGCKTGALMGCVGATATCHCSIHVSK (SEQ ID NO: 14);
ILSISLCTPGCKTGALMGCVGVTATCHCSIHVSK (SEQ ID NO: 15);
ILSISLCTPGCKTGALMGCVGTTATCHCSIHVSK (SEQ ID NO: 16);
ILSISLCTPGCKTGALQGCHTKTATCHCSIHVSK (SEQ ID NO: 17);
ILSISLCTPGCKTGALQGCVGATATCHCSIHVSK (SEQ ID NO: 18);
ILSISLCTPGCKTGALQGCVGVTATCHCSIHVSK (SEQ ID NO: 19); and
ILSISLCTPGCKTGALQGCVGTTATCHCSIHVSK (SEQ ID NO: 20). The modified residues are underlined.

The modified Nisin may be of any Nisin peptide. In one embodiment the Nisin that is modified is Nisin A. In one embodiment, the modification/substitution at a given amino acid position may be in any form of Nisin, for example wild-type Nisin A, U, U2, Z, F, Q, H, J, O or P. In one embodiment, the wild-type Nisin variant may be anyone selected from the group of variants comprising Nisin A, U, U2, Z, F, Q, H, J, O or P. In one embodiment, the wild-type Nisin variant may be anyone selected from the group of variants comprising Nisin A, U, U2, Z, F, Q, H, O or P. In one embodiment, the wild-type Nisin variant may be anyone selected from the group of variants comprising Nisin A, Z, F and Q. Suitably, the wild-type Nisin variant may be Nisin A. Suitably, the wild-type Nisin variant may be Nisin Z. Suitably, the wild-type Nisin variant may be Nisin F. Suitably, the wild-type Nisin variant may be Nisin Q. The skilled person will recognise that the exact amino acid number to modify may be different in an alternative wild-type Nisin variant than Nisin A according to SEQ ID NO: 1. It is expected that the equivalent amino acid for substitution may be readily identified, for example by a standard sequence alignment.

In one embodiment the variant nisin molecule may have at least 75% identity with any one of wild-type SEQ ID NOs: 21-30. In another embodiment the variant nisin molecule may have at least 80% identity with any one of wild-type SEQ ID NOs: 21-30. In another embodiment the variant nisin molecule may have at least 85% identity with any one of wild-type SEQ ID NOs: 21-30. In another embodiment the variant nisin molecule may have at least 90% identity with any one of wild-type SEQ ID NOs: 21-30. In another embodiment the variant nisin molecule may have at least 95% identity with any one of wild-type SEQ ID NOs: 21-30. In another embodiment the variant nisin molecule may have at least 98% identity with any one of wild-type SEQ ID NOs: 21-30. In another embodiment the variant nisin molecule may have at least 99% identity with any one of wild-type SEQ ID NOs: 21-30. In another embodiment the variant nisin molecule may have at least 99.5% identity with any one of wild-type SEQ ID NOs: 21-30.

In an embodiment wherein the wild-type Nisin variant, e.g. U, U2, Z, F, Q, H, J, O or P, already has one of the modifications according to the invention, the present invention may not include such a wild-type variant. In another embodiment wherein the wild-type Nisin variant, e.g. U, U2, Z, F, Q, H, J, O or P, already has one of the modifications according to the invention, the present invention may include modifying the wild-type variant with one or more modifications listed according to the invention herein that are not already naturally provided in the wild-type variant.

In one embodiment the variant nisin molecule may have at least 75% identity with any one of SEQ ID NOs: 2-20. In another embodiment the variant nisin molecule may have at least 80% identity with any one of SEQ ID NOs: 2-20. In another embodiment the variant nisin molecule may have at least 85% identity with any one of SEQ ID NOs: 2-20. In another embodiment the variant nisin molecule may have at least 90% identity with any one of SEQ ID NOs: 2-20. In another embodiment the variant nisin molecule may have at least 95% identity with any one of SEQ ID NOs: 2-20. In another embodiment the variant nisin molecule may have at least 98% identity with any one of SEQ ID NOs: 2-20. In another embodiment the variant nisin molecule may have at least 99% identity with any one of SEQ ID NOs: 2-20. In another embodiment the variant nisin molecule may have at least 99.5% identity with any one of SEQ ID NOs: 2-20.

In one embodiment the variant nisin molecule may have at least 75% identity with any one of SEQ ID NOs: 17-20. In another embodiment the variant nisin molecule may have at least 80% identity with any one of SEQ ID NOs: 17-20. In another embodiment the variant nisin molecule may have at least 85% identity with any one of SEQ ID NOs: 17-20. In another embodiment the variant nisin molecule may have at least 90% identity with any one of SEQ ID NOs: 17-20. In another embodiment the variant nisin molecule may have at least 95% identity with any one of SEQ ID NOs: 17-20. In another embodiment the variant nisin molecule may have at least 98% identity with any one of SEQ ID NOs: 17-20. In another embodiment the variant nisin molecule may have at least 99% identity with any one of SEQ ID NOs: 17-20. In another embodiment the variant nisin molecule may have at least 99.5% identity with any one of SEQ ID NOs: 17-20.

In one embodiment, the variant nisin may comprise modifications limited to no more than one modified amino acid residue, two or less modified amino acid residues, three or less modified amino acid residues, four or less modified amino acid residues, five or less modified amino acid residues, six or less modified amino acid residues, ten or less modified amino acid residues, 15 or less modified amino acid residues relative to any one of SEQ ID NOs: 2-20.

In one embodiment, the nisin variant comprises or consists of the sequence ILSISLCTPGCKTGALQGCHTKTATCHCSIHVSK (SEQ ID NO: 17).

In one embodiment, the variant nisin is about 34 amino acids in length. The variant nisin may be no more than 34 amino acids in length. In one embodiment, the variant nisin is a truncated variant of SEQ ID NO: 1, with the specified modifications. In another embodiment, the variant nisin is a truncated variant of any of SEQ ID NOs: 21-30, with the specified modifications. Alternatively, the variant nisin may be at least 34 amino acids in length. Alternatively, the variant nisin may be at least 33 amino acids in length. Alternatively, the variant nisin may be at least 32 amino acids in length. Alternatively, the variant nisin may be at least 31 amino acids in length. Alternatively, the variant nisin may be at least 30 amino acids in length. In one embodiment, the variant nisin is between about 20 and about 50 amino acids in length. In one embodiment, the variant nisin is between about 30 and about 50 amino acids in length. In one embodiment, the variant nisin is between about 34 and about 50 amino acids in length. In one embodiment, the variant nisin is between about 34 and about 40 amino acids in length. Reference to the amino acid length of the variant or wild-type nisin herein is not intended to include any leader sequence.

Single point mutations that have previously been shown to improve the potency of nisin A may further be provided as an additional Nisin modification. Such point mutations may be selected from I4V, K12A, K12T, K12S, K12P, A15G, M17S, N20P, N20K, N20E, M21A, M21V, M21K, K22A, K22T, K22S, K22G, H27Q, S29G, S29A, S29D, S29E, S29R, S29P, and H31K, or combinations thereof.

Additional hinge variants may also be provided in addition to the modifications of the invention, for example where NMK is replaced by one of PAL, PMQ, PTL, PMC, HLT, GVK. AAA, SAA, NAI, AAK, SLS, SMT, NAT, QVQ, PMA, PVN, SIN, ASS, ANP, SVA, PIT, PAQ, PGA, and PAK.

In one embodiment, for example in accordance with the first aspect, the variant nisin peptide is capable of inhibiting the growth of, or capable of killing, *Staphylococcus spp.,* such as *S. aureus* and/or *Streptococcus spp.,* such as *S. agalactiae.* Additionally or alternatively, for example in accordance with the first aspect, the variant nisin peptide may be capable of inhibiting the growth of, or capable of killing, one or more pathogenic bacteria selected from MRSA, VISA, hVISA, and *S. pseudintermedius.* In one embodiment, for example in accordance with the first aspect, the variant nisin peptide may be capable of inhibiting the growth of, or capable of killing, one or more pathogenic bacteria selected from *S. aureus, S. agalactiae, S. dysgalactiae, S. uberis,* MRSA, VISA, hVISA, and *S. pseudintermedius.*

In one embodiment, for example in accordance with the first aspect, the variant nisin peptide has higher potency for inhibiting the growth of, or killing, one or more pathogenic bacteria selected from *S. aureus, S. agalactiae, S. dysgalactiae, S. uberis,* MRSA, VISA, hVISA, and *S. pseudintermedius,* relative to wild-type nisin A. In one embodiment, for example in accordance with the first aspect, the variant nisin peptide has higher potency for inhibiting the growth of, or killing, *S. aureus* relative to wild-type nisin A.

The *S. aureus* may comprise the strain RF122, NCDO1499, 20777, or 54072. The *S. agalactiae* may comprise the strain ATCC13813.

In one embodiment, for example in accordance with the second aspect, the variant nisin peptide is capable of inhibiting the growth of, or capable of killing, *Thermus spp.* In one embodiment, for example in accordance with the second aspect, the variant nisin peptide has higher potency for inhibiting the growth of, or killing, *Thermus spp.* relative to wild-type nisin A.

The *Thermus spp.* may be any of the group comprising *T. thermophilus, T. scotoductus, T. rehai, T. oshimai, T. aquaticus* and *T. brockianus;* or combinations thereof. The *Thermus spp.* may be *T. thermophilus* and/or *T. scotoductus. The T. thermophilus* may be *T. thermophilus* HB27. The *T. scotoductus* may be *T. scotoductus* SE-1. The *Thermus spp.* may be any of the group comprising *T. rehai, T. oshimai, T. aquaticus* and *T. brockianus.* In another embodiment, the *Thermus spp.* comprises *T. thermophilus.* In another embodiment, the *Thermus spp.* comprises *T. scotoductus.* In another embodiment, the *Thermus spp.* comprises *T. rehai.* In another embodiment, the *Thermus spp.* comprises *T. oshimai.* In another embodiment, the *Thermus spp.* comprises *T. aquaticus.* In another embodiment, the *Thermus spp.* comprises *T. brockianus.*

The *T. rehai* may be *T. rehai* RH99-GF7504. The *T. oshimai* may be *T. oshimai* SPS-17. The *T. aquaticus* may be *T. aquaticus* YT-1. The *T. scotoductus* may be *T. scotoductus* GE-1.

In one embodiment, the higher potency may be at least 2-fold, 3-fold or 4-fold higher potency on average relative to wild-type nisin A. In another embodiment, the higher potency may be at least 10-fold, 20-fold or 30-fold higher potency on average relative to wild-type nisin A. The potency may be determined by the MIC of the peptide (minimum inhibitory concentration). The MIC may be 0.5 mg/L or less.

In one embodiment, the variant nisin peptide has lower potency for inhibiting the growth of, or killing, of lactic acid bacteria (LAB) relative to wild-type nisin A. The LAB may comprise lactococci and/or lactobacilli. The LAB may comprise *L. lactis,* such as *L. lactis* HP, *L. lactis* 303 or *L. lactis* MG1363. In one embodiment, the lower potency may be at least 2-fold, 3-fold or 4-fold lower potency on average relative to wild-type nisin A. In another embodiment, the lower potency may be at least 5-fold, 6-fold or 8-fold lower potency on average relative to wild-type nisin A.

In one embodiment, the variant nisin peptide has lower potency for inhibiting the growth of, or killing, of lactic acid bacteria (LAB) relative to wild-type nisin A.

According to another aspect of the invention, there is provided a nucleic acid encoding the variant nisin peptide according to the invention.

The nucleic acid may be in a vector, such as a viral vector. The nucleic acid may be a plasmid capable of replication in bacteria and/or yeast. For example the nucleic acid may comprise an origin of replication suitable for replication in bacteria and/or yeast, and/or plant cell. The nucleic acid may comprise a selection marker.

According to another aspect of the invention, there is provided a composition comprising the nucleic acid according to the invention.

### Nisin Composition

According to another aspect of the invention, there is provided a composition comprising the variant nisin according to the invention.

The amount of variant nisin peptide in the composition may be a therapeutically effective amount. For example, the amount of variant nisin peptide in the composition may be an amount that is sufficient to cause inhibition or killing of the target pathogenic bacteria, such as those pathogenic bacteria described herein.

The composition may further comprise one or more different variant nisin peptides, which may be variant nisin peptides according to the invention, wild-type nisins, or other known natural or synthetic variants/derivatives, or combinations thereof. For example, the composition may comprise two or more different variants of the variant nisin peptides according to the invention.

The composition may be a pharmaceutical composition. The composition may comprise a pharmaceutically acceptable carrier. The composition may further comprise one or more pharmaceutically acceptable excipients.

The carrier may comprise a buffer, such as a pharmaceutically acceptable buffer.

The composition may be suitable for topical administration to skin. The composition may be in the form of an ointment, cream/emulsion (o/w or w/o), paste, lotion, or gel, for example for topical administration.

The composition may be suitable for oral administration, for example for delivery into the gut. The composition may be in the form of a tablet, pill, liquid or gel that is suitable for oral administration. The composition suitable for oral administration may comprise one or more forms of variant nisin peptides according to the invention encapsulation within a polymeric material.

Advantageously, animal studies, e.g. involving chickens, mice, rats, guinea pigs etc. have shown that nisin can beneficially modulate the gut microbiota (Bernbom N., Appl Environ Microbiol. 2006 Jan;72(1):239-44).

The composition may be suitable for injection into the body of a subject, for example intravenous or sub-cutaneous injection. In one embodiment, the composition is suitable for intramammary infusion.

In one embodiment, the composition may be in the form of a teat seal. The composition may be a solid, for example after setting. In another embodiment, the composition may be a liquid that is arranged to set into a solid or semisolid, or set into a gel. In one embodiment, the composition may comprise paraffin and/or inorganic salt.

The skilled person will recognise that a teat seal is an oil-based formulation which forms a physical barrier against infection in the area of the teat canal and sinus. It may comprise an inorganic salt in a paraffin base.

The composition may be non-toxic to mammalian cells. The composition may be biologically compatible. The composition may be sterile. The composition may be non-immunogenic. In one embodiment, the composition may not comprise an ingredient that is toxic to, and/or is an irritant, to skin. The skilled person will understand that toxic or irritant properties of an ingredient may be in relation to "normal" (e.g. physiological or pharmaceutically acceptable) concentrations that may be typically used in such compositions.

The composition may comprise another active agent. For example, the composition may contain one or more steroids, antihistamines, sympathomimetics, beta receptor blockers, parasympathomimetics, parasympatholytics, prostaglandins, nonsteroidal anti-inflammatory drugs (NSAIDs), antibiotics, antifungal, or topical anaesthetics, or combinations thereof. In another embodiment, the composition may contain one or more steroids, antihistamines, sympathomimetics, beta receptor blockers, parasympathomimetics, parasympatholytics, prostaglandins, nonsteroidal anti-inflammatory drugs (NSAIDs), antifungal, or topical anaesthetics, or combinations thereof.

In one embodiment the composition may not comprise a non-bacteriocin antibiotic.

In one embodiment the composition is a liquid composition. For example the composition may comprise a solvent, such as water. In another embodiment, the composition may be in the form of a solid, for example a solid powder form. The solid may be arranged to be reconstituted in a solvent, such as water, prior to use.

In a further aspect of the invention there is provided a container comprising the composition according to the invention for treatment or prevention of mastitis.

The container may be in the form of an applicator device, such as a syringe, pipette, or dropper.

Wild-type Nisin is currently used in the form of Wipe Out® Dairy Wipes, a topical Nisin-based product used to prepare the cow's teat area prior to milking. Bayer have released Preva medicated wipes containing 25µg/ml nisin for topical use on dogs, cats, and horses with dermatological conditions associated with bacterial infections or general cleansing (www.animalhealth.bayerhealthcare.com).

Therefore, according to another aspect of the invention, there is provided a wipe impregnated with one or more forms of variant nisin peptides according to the invention.

The wipe may be in the form of a cloth or tissue. The wipe may comprise fibrous material. In one embodiment the wipe comprises porous material.

According to another aspect of the invention, there is provided a kit comprising:
- a nisin variant peptide according the invention or the composition according to the invention; and
- an applicator, such as a syringe, pipette, dropper, or wipe.

### Methods and uses for therapy

According to another aspect of the invention, there is provided a variant nisin peptide or composition according to the invention, for use as a medicament.

According to another aspect of the invention, there is provided a variant nisin peptide or composition according to the invention, for use in the treatment or prevention of a bacterial infection in a subject.

According to another aspect of the invention, there is provided a variant nisin peptide or composition according to the invention, for use in reducing bacterial colonisation of a subject.

According to another aspect of the invention, there is provided a method of treatment or prevention of a bacterial infection in a subject, wherein the method comprises the administration of the variant nisin peptide or composition according to the invention to the subject.

In one embodiment the bacterial infection is mastitis. In another embodiment the bacterial infection is a skin or wound infection.

In one embodiment, the mastitis or bacterial infection may be caused by infection by one or more pathogenic bacteria selected from *S. aureus, S. agalactiae, S. dysgalactiae, S. uberis,* MRSA, VISA, hVISA, and S. *pseudintermedius.* In one embodiment, the skin or wound infection or colonisation may comprise an *S. aureus* infection or colonisation, such as MRSA.

In one embodiment, the subject to be treated (or protected) may be mammalian. The subject to be treated (or protected) may be a livestock animal, such as a dairy livestock animal. The subject to be treated (or protected) may be a cow, sheep or goat. In one embodiment, the subject to be treated (or protected) is bovine. In one embodiment, the subject to be treated (or protected) may be human.

The subject may be pre-disposed to mastitis, or other bacterial infection. The subject may be a past or present mastitis, or other bacterial infection, sufferer. The subject may have re-occurring mastitis, or other bacterial infection. The subject may be a carrier of bacterial strains known to cause mastitis, or other bacterial infection.

The treatment may comprise the administered of the variant nisin peptide or composition according to the invention by injection or infusion into the subject. For example, an intramammary infusion, intramuscular injection or intravenous injection. In another embodiment, the administration may comprise a topical administration, for example of a cream or ointment. In another embodiment, the administration may comprise oral administration. In another embodiment, the administration may comprise the application of a teat seal comprising the variant nisin peptide or composition according to the invention.

The treatment may further comprise the administration or combined use with one or more other active therapeutic agents, such as a non-bacteriocin antibiotic and/or anti-inflammatory agent (such as a steroid).

The use of a Nisin variant according to the invention herein, or methods of the invention making use of the Nisin variant may also include the use of a wild-type variant that already has one or more of the described substitutions as a natural variation relative to Nisin A (SEQ ID NO: 1).

### Food and other use

According to another aspect of the invention, there is provided the use of the Nisin variant peptide, or composition, according to the invention to reduce or prevent bacterial contamination of a substrate, a liquid, a product, a surface, or equipment.

According to another aspect of the invention, there is provided the use of the Nisin variant peptide, or composition, according to the invention as a food preservative or additive.

According to another aspect of the invention, there is provided a method of preserving food against bacterial spoilage, (e.g. from *Thermus spp*.) the method comprising the addition of the Nisin variant peptide, or composition, according to the invention to one or more of the food, ingredients for food, food packaging and food preparation equipment.

Pink discolorations have been reported intermittently across a wide geographical area (e.g., Europe, USA, and New Zealand) in a range of ripened cheeses employing differing manufacturing processes, including Swiss, Cheddar, Grana, and Italian types. The defect results in the downgrading or rejection of cheese and a consequential economic loss. The Thermus bacterium has only recently been identified as the causative agent (Quigley et al., 2016), where a study showed that the microbe was found in the hot water supply pipes of each facility.

According to another aspect of the invention, there is provided the use of the Nisin variant peptide, or composition, according to the invention for reducing or inhibiting the growth of bacteria, such as *Thermus spp.,* in one or more of food, such as cheese, ingredients for food, food packaging, and food preparation equipment.

The use may be in a cheese starter culture and/or pasteurisation process. The use may be in a alcoholic fermentation process, such as brewing. The use may be in a food preservation process, such as canning or bottling of meat and/or vegetable food products.

The bacteria, such as the spoilage/contaminating bacteria, may comprise any one or more of the bacteria described herein.

The spoilage/contaminating bacteria may comprise any bacterial species that is inhibited or killed by the variant nisin according to the invention herein. The spoilage/contaminating bacteria may comprise a *Thermus spp.*

The *Thermus spp.* may be any of the group comprising *T. thermophilus, T. scotoductus, T. rehai, T. oshimai, T. aquaticus* and *T. brockianus;* or combinations thereof. The *Thermus spp.* may be *T. thermophilus* and/or *T. scotoductus. The T. thermophilus* may be *T. thermophilus* HB27. The *T. scotoductus* may be *T. scotoductus* SE-1. The *Thermus spp.* may be any of the group comprising *T. rehai, T. oshimai, T. aquaticus* and *T.* brockianus; or combinations thereof. The *Thermus spp.* may be any of the group comprising *T. rehai* RH99-GF7504, *T. oshimai* SPS-17, *T. aquaticus* YT-1 and *T. scotoductus* GE-1; or combinations thereof

The product may comprise a food product or an ingredient thereof, a beverage, medicinal product, cosmetic product, or personal hygiene product. The beverage may be a fermented alcoholic beverage.

The food may comprise cheese and/or milk, or milk-derived products. The cheese may comprise cheese spread. The food may comprise canned or bottled vegetable and/or meat.

The food ingredients may comprise water, such as hot water.

The food preparation equipment may be any tool, container, packaging, tubing/pipework, machinery, or pump system, that may come into contact with the food or food ingredients during the production process. In one embodiment, the food equipment comprises hot water supply equipment, such as hot water supply pipes and/or tanks/reservoirs. Such equipment may be immersed in, doused with, sprayed with, or wiped with, a composition comprising a nisin variant according to the invention herein. The packaging may comprise a can or bottle.

In another embodiment, the use may be in oral healthcare products such as toothpaste or mouthwash. Therefore, the composition according to the invention may comprise an oral healthcare composition, such as toothpaste or mouthwash. The skilled person will recognise that an oral healthcare composition may comprise one or more orally acceptable excipients (e.g. not harmful/toxic to mucous membranes at standard/acceptable concentrations).

In another embodiment, the use may be in topical compositions for the skin or mucous membranes. Therefore, the composition according to the invention may comprise a topical composition. The topical composition may comprise a topical composition for the treatment of a skin condition, such as acne. The topical composition may comprise a cream, ointment, or oil. The topical composition may be a cosmetic composition. Alternatively, the topical composition may be a prophylactic and/or therapeutic composition. The skilled person will recognise that a topical composition may comprise one or more topically acceptable excipients (e.g. not harmful/toxic to the skin at standard/acceptable concentrations).

The composition may comprise one or more components selected from polymer, humectant. fatty acid, lipid, protein, peptide, surfactant, alcohol, plant extract, CoQ10, clay, sugar, oil, glycerine, glycerol, sorbitol, hyaluronic acid, paraben, silicone, and metal oxide.

According to another aspect of the invention, there is provided the use of the Nisin variant peptide, or composition, according to the invention as preservative or additive in a healthcare or cosmetic product.

### Cell for Expression/production

According to another aspect of the invention, there is provided a cell that is capable of expressing one or more variant nisin peptides according to the invention herein.

The cell may be modified to express one or more variant nisin peptides according to the invention herein. The cell may comprise, or be modified by, the nucleic acid according to the invention. The modification may be a stable modification such that the nucleic acid according to the invention is integrated into the cell genome.

In one embodiment, the cell is a bacterial cell, such as a *Lactococcus spp.* In one embodiment, the cell comprises *L. lactis,* such as *L. lactis* NZ9800. In another embodiment, the cell is a eukaryote cell, such as a plant cell or yeast cell.

According to another aspect of the invention, there is provided a method of producing the variant nisin peptide according to the invention, the method comprising expressing variant nisin peptide in a cell culture that has been modified to produce the variant nisin peptide.

The method may comprise fermentation of the cells. The method may further comprise extraction of the variant nisin peptide from the cells and/or supernatant of the cell culture. The extraction may comprise isolation/purification of the variant nisin peptide. The method may comprise the step of extraction and purification to a pharmaceutical grade powder.

### Definitions

The term "treatment" used herein in the context of treatment of mastitis is understood to mean at least a partial reduction in mastitis, or other bacterial infection, symptoms and/or severity. In one embodiment, the treatment may be at least a 50% reduction in mastitis, or other bacterial infection, symptoms or severity. In one embodiment, the treatment may be at least a substantive or complete resolution of mastitis, or other bacterial infection, in a subject. In one embodiment, the treatment may comprise a reduction in inflammation and/or limiting potential tissue damage.

The term "prevention" used herein in the context of prevention of mastitis, or other bacterial infection, is understood to mean the reduction of incidence (e.g. frequency or length) and/or severity of mastitis, or other bacterial infection, in a subject.

The skilled person will appreciate that preferred features of any one embodiment and/or aspect of the invention may be applied to all other embodiments and/or aspects of the invention.

### Aspects of the invention

Aspects and embodiments of the present invention may be described in accordance with the following numbered paragraphs.
1. A variant nisin peptide, wherein the variant nisin peptide is a modified variant of wild-type nisin, wherein the modification comprises or consists of an amino acid residue modification selected from:
   i) M17Q or M17A;
   ii) T2L; and
   iii) one set of modified hinge residues selected from a) N20H and M21T, b) N20V, M21G, and K22A, c) N20V, M21G, and K22V or d) N20V, M21G, and K22T; or
   iv) combinations of modifications i), ii) and iii).
2. A variant nisin peptide, wherein the variant nisin peptide is a modified variant of wild-type nisin, wherein the modification comprises or consists of an amino acid residue modification selected from:
   i) M17Q;
   ii) K12T or K12V
   iii) N20S
   iv) one of M21F, M21A, M21V, M21Y, or M21Q;
   v) H27Q; and
   vi) one set of modified hinge residues selected from a) N20V, M21G, and K22A, or b) N20P, M21A, and H27Q; or
   vii) combinations of modifications i) to vi).
3. The variant nisin peptide according to paragraph 1, wherein the variant nisin peptide comprises or consists of any one of the sequences selected from the group comprising:
   ITSISLCTPGCKTGALQGCNMKTATCHCSIHVSK (SEQ ID NO: 2);
   ILSISLCTPGCKTGALMGCNMKTATCHCSIHVSK (SEQ ID NO: 3);
   ITSISLCTPGCKTGALMGCHTKTATCHCSIHVSK (SEQ ID NO: 4);
   ITSISLCTPGCKTGALMGCVGATATCHCSIHVSK (SEQ ID NO: 5);
   ITSISLCTPGCKTGALMGCVGVTATCHCSIHVSK (SEQ ID NO: 6);
   ITSISLCTPGCKTGALMGCVGTTATCHCSIHVSK (SEQ ID NO: 7);
   ILSISLCTPGCKTGALQGCNMKTATCHCSIHVSK (SEQ ID NO: 8);
   ITSISLCTPGCKTGALQGCHTKTATCHCSIHVSK (SEQ ID NO: 9);
   ITSISLCTPGCKTGALQGCVGATATCHCSIHVSK (SEQ ID NO: 10);
   ITSISLCTPGCKTGALQGCVGVTATCHCSIHVSK (SEQ ID NO: 11);
   ITSISLCTPGCKTGALQGCVGTTATCHCSIHVSK (SEQ ID NO: 12);
   ILSISLCTPGCKTGALMGCHTKTATCHCSIHVSK (SEQ ID NO: 13);
   ILSISLCTPGCKTGALMGCVGATATCHCSIHVSK (SEQ ID NO: 14);
   ILSISLCTPGCKTGALMGCVGVTATCHCSIHVSK (SEQ ID NO: 15);
   ILSISLCTPGCKTGALMGCVGTTATCHCSIHVSK (SEQ ID NO: 16);
   ILSISLCTPGCKTGALQGCHTKTATCHCSIHVSK (SEQ ID NO: 17);
   ILSISLCTPGCKTGALQGCVGATATCHCSIHVSK (SEQ ID NO: 18);
   ILSISLCTPGCKTGALQGCVGVTATCHCSIHVSK (SEQ ID NO: 19); and
   ILSISLCTPGCKTGALQGCVGTTATCHCSIHVSK (SEQ ID NO: 20); or variants thereof.
3. The variant nisin peptide according to any preceding paragraph, wherein the modified Nisin may be of any one of wild-type nisin peptides A, U, U2, Z, F, Q, H, J, O or P.
4. The variant nisin peptide according to any preceding paragraph, wherein the modification further comprises a modification selected from I4V, K12A, K12T, K12S, K12P, A15G, M17S, N20P, N20K, N20E, M21A, M21V, M21K, K22A, K22T, K22S, K22G, H27Q, S29G, S29A, S29D, S29E, S29R, S29P, and H31K, or combinations thereof.
5. The variant nisin peptide according to any preceding paragraph, wherein the modification further comprises a modification to S29 and/or 130.
6. The variant nisin peptide according to any preceding paragraph, wherein the modification further comprises a modification where NMK hinge residues are replaced by one of PAL, PMQ, PTL, PMC, HLT, GVK. AAA, SAA, NAI, AAK, SLS, SMT, NAT, QVQ, PMA, PVN, SIN, ASS, ANP, SVA, PIT, PAQ, PGA, and PAK.
7. The variant nisin peptide according to any of paragraphs 1 and 3-6, wherein the variant nisin peptide is capable of inhibiting the growth of, or capable of killing, *Staphylococcus spp.* and/or *Streptococcus spp.*
8. The variant nisin peptide according to any of paragraphs 1 and 3-6, wherein the variant nisin peptide is capable of inhibiting the growth of, or capable of killing, one or more pathogenic bacteria selected from *S. aureus, S. agalactiae, S. dysgalactiae, S. uberis,* MRSA, VISA, hVISA, and *S. pseudintermedius.*
9. The variant nisin peptide according to any of paragraphs 1 and 3-6, wherein the variant nisin peptide has higher potency for inhibiting the growth of, or killing, one or more pathogenic bacteria selected from *S. aureus, S. agalactiae, S. dysgalactiae, S. uberis,* MRSA, VISA, hVISA, and *S. pseudintermedius,* relative to wild-type nisin A.
10. The variant nisin peptide according to any of paragraphs 2-6, wherein the variant nisin peptide is capable of inhibiting the growth of, or capable of killing, *Thermus spp.*
11. The variant nisin peptide according to any of paragraphs 2-6, wherein the variant nisin peptide has higher potency for inhibiting the growth of, or killing, *Thermus spp.* relative to wild-type nisin A.
12. The variant nisin peptide according to any preceding paragraph, wherein the variant nisin peptide has lower potency for inhibiting the growth of, or killing, of lactic acid bacteria (LAB) relative to wild-type nisin A.
13. A nucleic acid encoding the variant nisin peptide according to any preceding paragraph.
14. A composition comprising a variant nisin according to any one of paragraphs 1-12, or comprising the nucleic acid according to paragraph 13.
15. The compositing according to paragraph 14, wherein the composition is a pharmaceutical composition comprising a pharmaceutically acceptable carrier.
16. The compositing according to paragraph 14 or 15, wherein the composition is suitable for topical administration to skin, oral administration, injection, or intramammary infusion.
17. The compositing according to any one of paragraphs 14-16, wherein the composition is in the form of an ointment, cream/emulsion, paste, lotion, or gel.
18. The compositing according to any one of paragraphs 14-17, wherein the composition is in the form of a teat seal.
19. The compositing according to any one of paragraphs 14-18, wherein the composition comprises paraffin and/or inorganic salt.
20. A container comprising the composition according to any one of paragraphs 14-19 for treatment or prevention of mastitis.
21. The container according to paragraph 20, wherein the container is a syringe, pipette, or dropper.
22. A wipe impregnated with one or more forms of variant nisin peptides according to any one of paragraphs 1-12, or the composition according to any one of paragraphs 14-19.
23. A kit comprising:
   - a nisin variant peptide according to any one of paragraphs 1-12, or the composition according to any one of paragraphs 14-19; and
   - an applicator, such as a syringe, pipette, dropper, or wipe.
24. A variant nisin according to any one of paragraphs 1-12, or the composition according to any one of paragraphs 14-19, for use as a medicament.
25. A variant nisin peptide according to any one of paragraphs 1-12, or the composition according to any one of paragraphs 14-19, for use in the treatment or prevention of a bacterial infection in a subject.
26. A variant nisin peptide according to any one of paragraphs 1-12, or the composition according to any one of paragraphs 14-19, for use in reducing bacterial colonisation of a subject.
27. A method of treatment or prevention of a bacterial infection in a subject, wherein the method comprises the administration of the variant nisin peptide according to any one of paragraphs 1-12, or the composition according to any one of paragraphs 14-19, to the subject.
28. The use of the Nisin variant peptide according to any one of paragraphs 1-12, or the composition according to any one of paragraphs 14-19, as a food preservative.
29. A method of preserving food against spoilage from *Thermus spp.* the method comprising the addition of the Nisin variant peptide according to any one of paragraphs 1-12, or the composition according to any one of paragraphs 14-19, to one or more of the food, ingredients for food, and food preparation equipment.
30. The use according to paragraph 28, or the method according to paragraph 29, wherein the food comprises or consists of cheese.
31. Use of the nisin variant peptide according to any one of paragraphs 1-12, or the composition according to any one of paragraphs 14-19, for reducing or inhibiting the growth of *Thermus spp.* in one or more of food, ingredients for food, and food preparation equipment.
32. A cell that is capable of expressing one or more variant nisin peptides according to any one of paragraphs 1-12, or comprises the nucleic acid according to paragraph 13.
33. A method of producing the variant nisin peptide according to any one of paragraphs 1-12, the method comprising expressing variant nisin peptide in a cell culture that has been modified to produce the variant nisin peptide.

Embodiments of the invention will now be described in more detail, by way of example only, with reference to the accompanying figures.

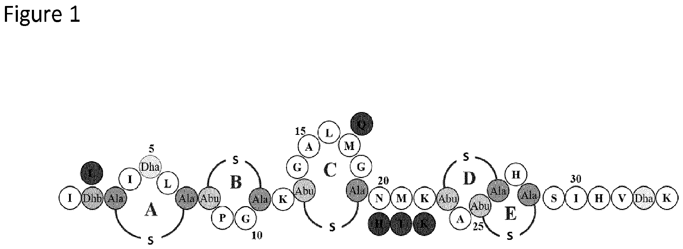

**Figure 1****.** Structure of nisin A. Residues are represented in the single letter code. Post translational modifications are indicated as follows, Dha: dehydroalanine, Dhb: dehydrobutyrine, Abu: 2-aminobutyric acid, Ala-S-Ala: lanthionine, Abu-S-Ala: 3-methyllanthionine. Amino acid substitutions T2L, M17Q and HTK are highlighted in red.

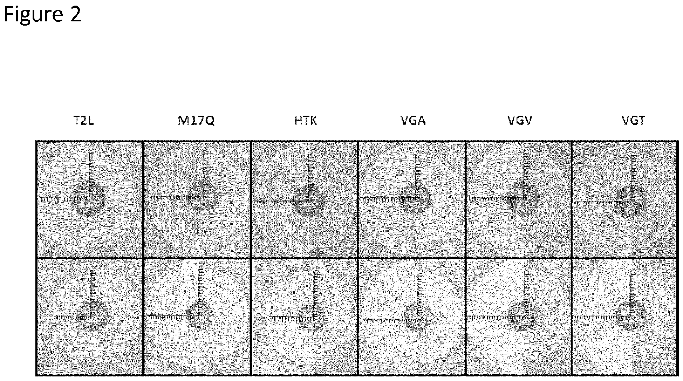

**Figure 2****.** Deferred antagonism assays of nisin derivatives against the nisin-sensitive indicators (top row) *S. aureus* RF122 and *Lactocococcus lactis* HP (bottom row). Each zone is comprised of the derivative producing strain on the left and for comparison the wild type producing strain to the right. Not to scale.

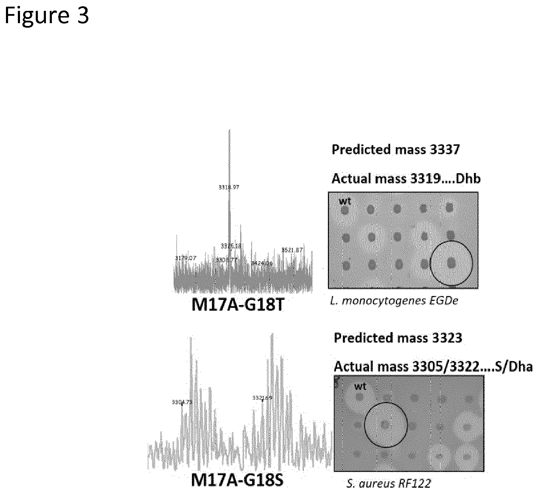

**Figure 3** **-** Deferred antagonism assays of the nisin A combination variants M17A-G18T against *L. monocytogenes* EGDe (top right circled) and M17A-G18S against *S. aureus* RF122 (bottom right circled; wt = wild type control). Mass spectrometry analysis of the *L. lactis* M17A-G18T producer strain (top left) reveals production of a peptide corresponding to M17A-G18Dhb (threonine is modified to dehydrobutyrine; 3319 Da). In the case of *L. lactis* M17A-G18S, two variant forms are produced whereby serine is both modified (G18Dha = Dehydroalanine; 3305 Da) and remains unmodified (3322 Da).

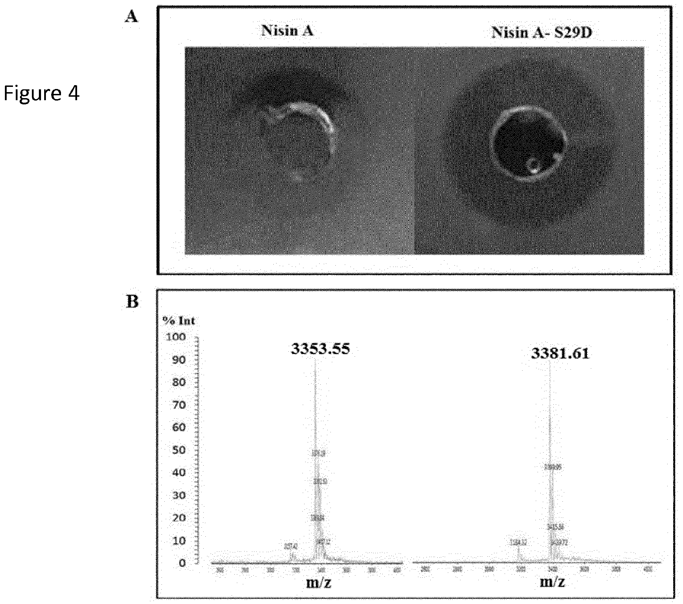

**Figure 4****: Bioactivity and mass spectrometry analysis of nisin A and nisin S29D.** A) Growth inhibition of *T. scotoductus* SE-1 by nisin A producing *L. lactis* NZ9700 and its bioengineered variant S29D producing *L. lactis* NZ9700. B) MALDI TOF analysis of purified peptides, nisin A (3353.55 amu) and nisin A- S29D (3381.61 amu).

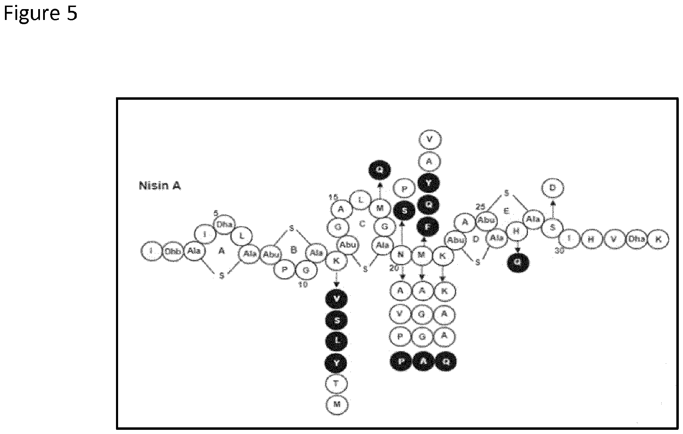

**Figure 5****: Nisin A peptide linear structure and bioengineered variants.** Arrow denotes the single and combination (joined circles) of amino acids substitution at a certain positions. Black circle indicate enhanced specific activity of that bioengineered variant

### Example 1 - Adapting the specific activity of nisin to control bovine mastitis

### Materials and Methods

### Bacterial Strains and Growth Conditions

*L. lactis* strains were grown in M17 broth supplemented with 0.5% glucose (GM17) or GM17 agar at 30°C. Bifidobacteria were grown anaerobically at 37°C in Reinforced Clostridial Medium (RCM) or RCM agar (Oxoid), lactobacillus strains were grown in De Man Rogosa and Sharpe (MRS) or MRS agar anaerobically at 37°C . *Escherichia coli* was grown in Luria-Bertani broth with vigorous shaking or agar at 37°C. *Staphylococcus* and *Streptococcus* strains were grown in Brain Heart Infusion (BHI)(Oxoid) or Tryptic Soy Broth (TSB) (Merck) at 37°C. Antibiotics were used where indicated at the following concentrations: Chloramphenicol at 10 and 20 µg ml⁻¹ for *L. lactis* and *E. coli,* respectively.

### Creation and screening a bank of nisin derivatives

Mutagenesis of the *nis*A gene was carried out as described previously (Field *et al.,* 2008). Briefly, saturation mutagenesis was carried out using pDF05 (pCI372-*nisA*) as template and using oligonucleotides containing an NNK codon in place of each native codon. PCR amplification was performed in a 50 µl reaction containing approximately 0.5 ng of target DNA (pDF05), 1 unit Phusion High-Fidelity DNA polymerase (Finnzymes, Finland), 1 mM dNTPs and 500 ng each of the appropriate forward and reverse oligonucleotide. The reaction was pre-heated at 98°C for 2 min, and then incubated for 29 cycles at 98°C for 30 s, 55°C for 15 s and 72°C for 3 min 30 s, and then finished by incubating at 72°C for 3 min 30 s. Amplified products were treated with Dpn1 (Stratagene) for 60 min at 37°C to digest template DNA and purified using the QIAquick PCR purification kit. Following transformation of *E. coli* Top 10 cells plasmid DNA was isolated and sequenced using primers pCI372FOR (5' CGGGAAGCTAGAGTAAGTAG 3') and pCI372REV (5' ACCTCTCGGTTATGAGTTAG 3') to verify that mutagenesis had taken place. Approximately 150 transformants were chosen at random for each position and inoculated into 96-well plates containing GM17 chloramphenicol, incubated overnight and stored at -20°C after addition of 80% glycerol. Deferred antagonism assays were performed by replicating strains on GM17 agar plates and allowing them to grow overnight before overlaying with BHI agar (0.75% w/v agar) seeded with the appropriate indicator strain.

### Mass Spectrometry

For Colony Mass Spectrometry (CMS) bacteria were collected with sterile plastic loops and mixed with 50 µl of 70% isopropanol adjusted to pH 2 with HCl. The suspension was vortexed, the cells spun down in a benchtop centrifuge at 14,000 r.p.m. for 2 min, and the supernatant was removed for analysis. Mass Spectrometry in all cases was performed with an Axima CFR plus MALDI TOF mass spectrometer (Shimadzu Biotech, Manchester, UK). A 0.5µl aliquot of matrix solution (alpha-cyano-4-hydroxy cinnamic acid (CHCA), 10 mg ml⁻¹ in 50% acetonitrile-0.1% (v/v) trifluoroacetic acid) was placed onto the target and left for 1-2 min before being removed. The residual solution was then air dried and the sample solution (resuspended lyophilised powder or CMS supernatant) was positioned onto the precoated sample spot. Matrix solution (0.5µl) was added to the sample and allowed to air-dry. The sample was subsequently analysed in positive-ion reflectron mode.

### Nisin purification

2 litres of Tryptone Yeast (TY) broth were incubated for 20 hours with 1% inoculum of an overnight culture of producing strain. This culture was centrifuged for 20 minutes @ 7000rpm. The supernatant was decanted and passed through 60 g of pre equilibrated Amberlite XAD16 beads (Sigma-Aldrich). The beads were washed with 500 ml 30% ethanol and eluted with 500 ml 70% isopropanol (IPA) (Fisher) 0.1% trifluoroacetic acid (TFA) (Sigma-Aldrich). Concomitantly, the cell pellets were resuspended in 300 ml of 70% IPA 0.1%TFA and stirred at room temperature for 3 hours followed by centrifugation. This cell supernatant was combined with that referred to above and concentrated through rotary-evaporation (Buchi, Switzerland) to approximately 250 ml. Following pH adjustment to 4.0 further concentration was achieved through the use of a Phenomenex SPEC-18 column to a final volume of 60 ml. 8 ml of this sample was concentrated, through rotary evaporation, to 2 ml and purified through HPLC using a Phenomenex C12 Reverse-Phase (RP) HPLC column (Jupiter 4 µ proteo 90 Å, 250 X 10.0 mm, 4 µm). To facilitate this, a gradient of 30-50% acetonitrile (Fisher) containing 0.1% TFA was developed. The relevant fractions were collected and pooled, subjected to rotary-evaporation to remove acetonitrile and freeze-dried (LABCONCO). The purified peptides were subjected to MALDI-ToF Mass Spectrometric analysis to confirm their purity before use.

### Minimum Inhibitory Concentration assays

Minimum inhibitory concentration determinations were carried out in triplicate in 96 well microtitre plates. 96 well microtitre plates were pre-treated with bovine serum albumin (BSA) prior to addition of the peptides. Briefly, to each well of the microtitre plate 200 µL of phosphate buffered saline (PBS) containing 1% (w/v) bovine serum albumin (PBS/BSA) was added and incubated at 37°C for 30 min. The wells were washed with 200 µL PBS and allowed to dry. Target strains were grown overnight in the appropriate conditions and medium, subcultured into fresh broth and allowed to grow to an OD₆₀₀ of ∼0.5, diluted to a final concentration of 10⁵ cfu ml⁻¹ in a volume of 0.2 ml. The lyophilised peptides were resuspended in the appropriate media to a stock concentration of 30 µM. Wild type nisin and nisin mutant peptides were adjusted to a 7.5 or 5.0 µM or 500 nM starting concentration and 2-fold serial dilutions of each peptide were made in 96 well plates for a total of 12 dilutions. The target strain was then added and after incubation for 16 h at 30°C or 37°C the MIC was read as the lowest peptide concentration causing inhibition of visible growth.

### Results

### Identification of nisin derivatives with enhanced bioactivity against S. aureus strains associated with bovine mastitis.

Previously, site-saturation mutagenesis-based strategies were used to generate strains producing bioengineered nisin derivatives in which all residues not involved in lanthionine ring formation (i.e. I1, T2, 14, S5, L6, P9, G10, K12, G14, A15, L16, M17, G18, N20, M21, K22, A24, H27, S29 130, H31, V32, S33 and K34) were each randomised to potentially all other natural amino acids, or whereby all three residues of the hinge-region (NMK) were randomised simultaneously (Rouse *et al.,* 2012, Healy *et al.,* 2013, Field *et al.,* 2008). In this study, the combined banks of approximately 30,000 individual producers were screened using deferred antagonism agar diffusion assays to identify those which display bioactivity (as assessed by zones of clearing) that, was greater than that of the nisin A producing control against mastitis-associated staphylococci, i.e. *S. aureus* RF122, *S. aureus* NCDO1499, and streptococci (*S. agalactiae* ATCC13813) but was relatively reduced against the LAB targets *L. lactis* HP and *L. lactis* MG1363. From this screen 76 variants of interest from the first round of screening were incorporated into a "mini-bank" for subsequent further analysis. The number of indicator strains was increased from five to thirteen to include *S. uberis* 43382, *S. uberis* DPC5344, *S. dysgalactiae* ATCC43078, *L. lactis* 303, *L. cremoris* KH, *Lactobacillus bulgaricus* NCDO2489, *Lb. helveticus* 2079 and *Lb. acidophilus* ATCC4356 (Table 1). Following this second round of screening, 6 variants of interest were deemed worthy of closer inspection. DNA sequencing of the mutated nisin gene as well as mass spectrometric analysis of the peptide produced by each strain established that these corresponded to nisin M17Q (methionine at position 17 altered to glutamine), nisin T2L (threonine 2 leucine) nisin HTK, VGA, VGV or VGT (where the hinge residues Asn-Met-Lys have been altered to His-Thr-Lys, Val-Gly-Ala, Val-Gly-Val or Val-Gly-Thr respectively). Strains producing the variants nisin VGA, VGV and VGT exhibited enhanced activity towards the majority of but not all of the mastitis-associated target strains of *S. aureus, S. uberis, S agalactiae and S. dysgalactiae,* but were also greatly enhanced against the lactococcal targets (Table 2; Figure 2). As a result, these derivatives were eliminated from this study, but will be the subject of further investigation as potential anti-mastitis antimicrobials. Nisin M17Q was notable by virtue of the fact that it displayed the greatest bioactivity towards the pathogenic indicators compared to all other nisin variants of this study, although this enhanced activity was also apparent against the lactococcal strains (Table 2). In the case of the derivatives nisin T2L and nisin HTK, the activity appeared reduced and equivalent to nisin A respectively (Figure 1), while improved activity was observed by both derivatives against *S. agalactiae* ATCC13813 and *S. aureus* RF122 (Table 2). On this basis, the nisin derivatives M17Q, T2L and HTK were selected for purification and specific activity assays. Purified peptides were subjected to Mass Spectrometry to confirm the correct mass in each case. In the case of T2L and HTK derivatives, the threonine remained in the unmodified form (data not shown).

### MIC-based investigations demonstrate enhanced specific activity of nisin derivatives against bovine mastitis-associated S. aureus

To confirm that the observed enhanced activity of the nisin variants was due to increased specific activity, the activity of purified peptides were assessed using classical broth-based minimum inhibitory concentration (MIC) determination assays. Following high performance liquid chromatography (HPLC) and freeze-drying to obtain purified peptide, MIC assays were carried out using equimolar concentrations of wild type nisin A, nisin A M17Q, nisin A T2L, and the hinge variant nisin A HTK against a range of targets including 12 *S*. *aureus* isolates comprising bovine-mastitis associated strains RF122, NCDO1499, 20777, 54072, DPC 5243, DPC 5245, DPC 5247, as well as *S. aureus* isolates 5971, 513, 272, and 2 isolates of methicillin-resistant *S. aureus* (MRSA) ST 528 and ST 534. Also included were the lactic acid bacteria targets *L. lactis* MG1363, *L. lactis* spp. *lactis* HP, *B. longum* UCC44b and *Lb acidophilus* ATCC 4356. The MIC was determined to be the lowest concentration of peptide that resulted in the absence of visible growth of the target strain after 16 hours at 37°C. All 3 nisin derivatives displayed variable activities against the panel of *S. aureus* strains tested. Nisin M17Q and nisin HTK displayed enhanced specific activity against 6 of the *S. aureus* strains utilised in the study. Mean MIC values for nisin A against *S. aureus* NCDO1499, *S. aureus* 54072, *S. aureus* RF122 and *S*. *aureus* 20777 were 0.25, 1, 2, and 4 mg/L, respectively (Table 2). In contrast, the corresponding nisin M17Q MIC values were 0.06, 0.25, 0.25 and 0.5 mg/L, respectively, representing a 4-16 fold increase in potency compared to the wild-type peptide. The nisin HTK derivative also displayed superior potency compared to nisin A against *S. aureus* NCDO1499 (0.03 mg/L), *S. aureus* 54072 (0.25 mg/L), and *S. aureus* 20777 (0.125 mg/L) and *S. aureus* RF122 (1 Mg/L). Lastly, although nisin T2L exhibited reduced specific activity against *S*. *aureus* NCDO1499 (1 mg/L) and against *S. aureus* 54072 (4 mg/L) (Table 2), it displayed improved activity against 9 of the remaining *S. aureus* strains utilised. Notably, T2L exhibited 4-fold enhanced activity against *S. aureus* 272, ST528 and 534 (MRSA) and displayed 16-fold improved activity against *S*. *aureus* 5971 (Table. 2).

When the lactococcal targets were assessed, all nisin variants exhibited a 2 fold reduced specific activity against both *L. lactis* strains. The MIC for nisin HTK, M17Q and T2L against *L. lactis* MG1363 was 0.4, 0.4 and 0.4mg/L, respectively, compared to a wild-type nisin A value of 0.2 mg/L. Similarly, nisin HTK, M17Q and T2L displayed a 2 fold reduction in potency against *L*. *lactis* HP(Table 2). When assessed against the target strain *B. longum* UCC 44b, Both HTK and T2L displayed 4-fold (0.24 mg/L) and 2-fold (0.12 mg/L) decreased activity respectively compared to wild type peptide (0.06 mg/L, while M17Q displayed equal activity to nisin A. Finally, when *Lb. acidophilus* ATCC 4356 was employed as the target, nisin HTK and T2L exhibited a 4-fold reduction (0.12 mg/L) in specific activity compared to wild type nisin A (0.03 mg/L) and in this case M17Q was 2-fold less active (0.06 mg/L).

### Discussion

The economic significance of mastitis as a persistent disease in dairy farming coupled with the emergence of antibiotic resistance in pathogenic bacteria has led to research into the most advantageous treatment strategies for current antimicrobial agents as well as a search for new alternatives to conventional therapy. Due to its existing broad spectrum activity against a wide range of targets, and its gene-encoded nature, we view nisin A as an excellent target for bioengineering-based development to generate more potent microbial inhibitors. Indeed, the identification of bioengineered nisin and other lantibiotic peptide derivatives with improved activity is becoming a more frequent event as a result of the creation of larger banks of engineered peptides. In this study, we undertook the largest screen of such peptides to date involving approximately 30,000 nisin derivatives and several representative mastitis-associated pathogenic targets including staphylococci and streptococci. We also set ourselves the more difficult task of uncovering novel derivatives with two distinct functional properties, namely to demonstrate enhanced activity towards pathogenic targets and show reduced activity towards many of the 'friendly' organisms naturally present in milk, i.e. lactococci and lactobacilli.In this regard we were successful in that three new nisin variants, nisin M17Q, nisin T2L and nisin HTK, exhibited these characteristics to varying extents. Notably, derivatives at threonine 2 and methionine 17 provided the first evidence that nisin could be enhanced against particular targets when nisin Z T2S, and nisin Z M17Q/G18T demonstrated increased activity against a limited number of non-pathogenic indicator strains (*M. flavus, S. thermophilus*)(Kuipers *et al.,* 1992).

The contrasting sensitivity of mastitis-associated *S. aureus* and industrially-relevant lactococci to nisin M17Q, T2L and HTK provides further evidence that some bioengineered nisin derivatives exhibit target specific variations in potency. Indeed, the variation in specific activities may reflect the enormous wealth and lineages of *S. aureus* strains that differ markedly with regard to pleiotropic transcriptional regulators and the numerous two-component systems (TCS) that act as a sophisticated arsenal of environmental monitoring proteins (Hiron *et al.,* 2011). For example, BraRS, the BceRS-likeTCS associated with *S. aureus,* has been shown to be essential for resistance to bacitracin, nisin, and nukacin ISK-1 and functions to reprogram gene expression to modify cell envelope architecture, facilitating adaptation and survival. Recently, it has been demonstrated that resistance to nisin arises readily in *Staphylococcus aureus* as a consequence of mutations in the *nsa*S gene, which encodes the sensor kinase of the NsaRS two-component regulatory system. Such systems in *S. aureus* constitute a potential threat to its efficacy in the treatment of bovine mastitis and represents an important factor to be considered in the context of advancing nisin and related bacteriocins toward therapeutic exploitation.

It has been suggested that a potential route to overcoming this resistance liability could involve modification of nisin to prevent such systems in recognizing it as a substrate. Indeed, a recent study involving high resolution NMR studies of nisin in cellular membranes identified plastic domains that enable the lantibiotic to adapt to the cellular environment (Medeiros-Silva *et al.,* 2018). Intriguingly, these plastic domains corresponded to pharmaceutical hotspots identified previously, including Ile 4 (Field *et al.,* 2015), Asn20-Met21-Lys22 (hinge region) (Field *et al.,* 2008, Healy *et al.,* 2013) Lys 12 (Molloy *et al.,* 2013) and Ser 29 (Field *et al.,* 2012) and enabled nisin's activity to be enhanced, establishing a link between antimicrobial activity and cellular adaptability. The HTK peptide represents a new enhanced hinge derivative to add to those previously identified through simultaneous, indiscriminate site-saturation mutagenesis of all three hinge residues, namely AAK, NAI and SLS, that displayed enhanced bioactivity against a variety of targets (Healy *et al.,* 2013). Notably however T2L and M17Q represent new enhanced derivatives whose locations have not previously been identified as 'hotspots' by NMR studies. This highlights the advantages of more methodical and rational screening strategies that generate molecular diversity through random and site saturation mutagenesis, followed by identification of library members with improvements by high-throughput screening or selection. In addition, the vast measure of data generated could potentially be evaluated through multidisciplinary approaches including Quantum Mechanical and Molecular Dynamics Simulations as well as machine-learning algorithms to effectively explore the impact of amino acid substitutions on lantibiotic peptide structure and stability. For example, recent molecular dynamics simulations combined with microbiological techniques provided valuable insights at the atomic level into the interactions of nisin and an improved nisin derivative in association with the nisin resistance protein (Field *et al.,* 2019).

The use of HTK, M17Q and T2L peptides as therapeutics for the treatment of mastitis would have many benefits given that milk LAB, but natural (nonstarter; NSLAB) and introduced (starter cultures and adjuncts), are key to the subsequent generation of fermented dairy products. LAB bring about the fermentation of milk through the production of lactate and have a variety of different impacts on the sensory, texture, flavour and organoleptic properties of resultant products. In addition, in contrast to antibiotics, the presence of nisin residues in milk may not require that the milk be withheld as nisin, being a ribosomal peptide, is susceptible to digestive enzymes and is easily destroyed in the gut. Notably, Immucell, who incorporate nisin into WipeOut® and MastOut®, teat wipe and intramammary infusion products respectively and used as therapeutics in the prevention of mastitis were granted a zero milk discard and zero meat withhold designation by the FDA (Roy *et al.,* 2016).

From a human medicine perspective, the potential of nisin in treating infectious mastitis in lactating mothers has already been demonstrated (Fernandez *et al.,* 2008). Significantly, nisin prepared from the Nis+ strain *L. lactis* ESI 515 effectively reduced staphylococcal numbers in breast milk and led to the complete disappearance of clinical signs of mastitis after two weeks of treatment. More importantly, nisin was successful where therapy using traditional antibiotics had failed to provide any improvement in symptoms (Fernandez *et al.,* 2008). Nisin A is already employed commercially as an anti-mastitis product in the form of Wipe Out® and Mast Out® in the veterinary field. As noted above, it has been suggested that Mast Out® may qualify for use in the US without a requirement to discard milk or withhold meat from human consumption as a consequence of treatment. Indeed, given the potency of nisin T2L, nisin M17Q, and nisin HTK against the mastitis-associated staphylococci of this study, it would seem that these peptides merit further attention as novel anti-mastitis antimicrobials.

### Wild-type Nisin SEQ ID NO: 1 (without leader sequence)

ITSISLCTPGCKTGALMGCNMKTATCHCSIHVSK

**Table 1. Strains utilized in this study**

| **Strains** | **Relevant characteristics** | **Reference** |
|---|---|---|
| ***L. lactis* NZ9800** | *L. lactis* NZ9700Δ*nis*A | (Kuipers *et al.,* 1998, Kuipers *et al.,* 1993) |
| ***L. lactis* NZ9800 pDF05** | *L. lactis* NZ9700Δ*nis*A with pCI372-nisA (pDF05) | |
| ***E. coli* Top 10** | Cloning host | Invitrogen |
| | | |

| **Indicator organisms** | | |
|---|---|---|
| ***S. aureus* 20777** | Mastitis-associated indicator strain | |
| ***S. aureus* NCD01499** | Mastitis-associated indicator strain | UCC culture collection |
| ***S. aureus* RF122** | Mastitis-associated indicator strain | UCC culture collection |
| ***Strep. uberis* 43382** | Mastitis-associated indicator strain | UCC culture collection |
| ***Strep. uberis* DPC 5344** | Mastitis-associated indicator strain | UCC culture collection |
| ***Strep. dysgalactiae* ATCC43078** | Mastitis-associated indicator strain | American Type Culture Collection |
| ***Strep. agalactiae* ATCC13813** | Mastitis-associated indicator strain | American Type Culture Collection |
| ***L. lactis sp cremoris* HP** | Starter culture | UCC culture collection |
| ***L. lactis MG*1363** | Prototype for LAB genetics | Gasson et al., 1983 |
| ***L. lactis 303* (DPC4268)** | Starter culture | UCC culture collection |
| ***L. lactis KH* (DPC745)** | Starter culture | UCC culture collection |
| ***Lb bulgaricus* NCDO2489** | Starter culture | UCC culture collection |
| ***Lb. helveticus* 2079** | Starter culture | UCC culture collection |
| ***Lb. acidophilus* ATCC4356** | Starter culture | American Type Culture Collection |
| ***B. longum* 44b** | | UCC culture collection |

**Table 2. Deferred antagonism results against representative bovine-mastitis associated strains and dairy lactococci strains. The zone of inhibition is expressed as the area of the zone of inhibition minus the area of the spot in mm2.**

| | **Nisin A** | **M17Q** | **T2L** | **HTK** | **VGA** | **VGV** | **VGT** |
|---|---|---|---|---|---|---|---|
| *L. lactis* HP | 230.60 ± 8.19 | **481.77 ± 8.95** | 134.12 ± 19.00 | 230.58 ± 6.71 | **362.16 ± 6.77** | **355.71 ± 11.03** | **353.67 ± 22.65** |
| *L. lactis* MG1363 | 149.28 ± 10.80 | **240.64 ± 12.96** | 80.11 ± 12.18 | 137.70 ± 5.40 | **230.92 ± 4.04** | **231.31 ± 7.80** | **214.53 ± 21.36** |
| *S*. *agalactiae* ATCC13813 | 147.16 ± 6.47 | **298.87 ± 6.87** | **194.44 ± 6.22** | **148.14 ± 3.66** | **234.96 ± 25.45** | **280.38 ± 8.10** | **245.42 ± 12.78** |
| *S*. *aureus* RF122 | 81.55 ± 5.31 | **136.92 ± 26.48** | 100.05 ± 14.96 | **106.66 ± 3.00** | **138.17 ± 8.12** | **121.15 ± 1.40** | **115.75 ± 2.65** |
| | | | | | | | |

**Table 3. Specific activity of nisin A and nisin M17Q, nisin T2L and nisin HTK against a range of indicator organisms. Underlined values denote a favourable outcome where specific activity is enhanced against staphylococci and reduced against a selection of lactic acid bacteria. *= denotes unfavourable outcome.**

| | **Nisin A Mg/L (µM)** | **HTK Mg/L (µM)** | **M17Q Mg/L (µM)** | **T2L Mg/L (µM)** |
|---|---|---|---|---|
| ***S. aureus* 20777** | 4 (1.25) | **0.4 (0.125)** | **0.5(0.156)** | **1 (0.312)** |
| ***S. aureus* 54072** | 1(0.312) | **0.25 (0.078)** | **0.25(0.078)** | 4 (1.25)* |
| ***S*. *aureus* NCDO1499** | 0.25 (0.078) | **0.06(0.019)** | **0.06(0.019)** | 1 (0.312)* |
| ***S. aureus* RF122** | 2 (0.625) | **1 (0.312)** | **0.25 (0.078)** | 2 (0.625)* |
| ***S. aureus* DPC 5243** | 1 (0.312) | 1 (0.312) | 2 (0.625)* | **0.5 (0.156)** |
| ***S. aureus* DPC 5245** | 1 (0.312) | 1 (0.312) | 1 (0.312)* | **0.5(0.156)** |
| ***S. aureus* DPC 5247** | 1 (0.312) | 1 (0.312) | 1 (0.312) | **0.5(0.156)** |
| ***S. aureus* 5971** | 25 (7.5) | **12.57 (3.75)** | **12.57 (3.75)** | **1.5 (0.468)** |
| ***S. aureus* 513** | 6 (1.875) | 6 (1.875) | 6 (1.875) | **3 (0.937)** |
| ***S. aureus* 272** | 3 (0.937) | 6 (1.875)* | **1.5 (0.468)** | **0.75 (0.234)** |
| ***S*. *aureus* ST528(MRSA)** | 0.5 (0.156) | **0.25 (0.078)** | **0.25 (0.078)** | **0.125(0.039)** |
| ***S*. *aureus* ST534(MRSA)** | 1 (0.312) | 1 (0.312) | 1 (0.312) | **0.25 (0.078)** |
| ***L. lactis* MG1363** | 0.2 (0.06) | **0.4 (0.125)** | **0.4 (0.125)** | **0.4(0.125)** |
| ***L. lactis* HP** | 0.2 (0.06) | **0.4 (0.125)** | **0.4 (0.125)** | **0.4(0.125)** |
| ***B. longum* UCC 44b** | 0.06(0.019) | **0.24(0.062)** | 0.06(0.019) | **0.12(0.030** |
| ***Lb. acidophilus ATCC 4356*** | 0.03(0.010) | **0.12(0.044)** | **0.06(0.022)** | **0.12(0.044)** |

### References

Cao, L.T., J.Q. Wu, F. Xie, S.H. Hu & Y. Mo, (2007) Efficacy of nisin in treatment of clinical mastitis in lactating dairy cows. J Dairy Sci 90: 3980-3985.
Fernandez, L., S. Delgado, H. Herrero, A. Maldonado & J.M. Rodriguez, (2008) The bacteriocin nisin, an effective agent for the treatment of staphylococcal mastitis during lactation. J Hum Lact 24: 311-316.
Field, D., M. Begley, P.M. O'Connor, K.M. Daly, F. Hugenholtz, P.D. Cotter, C. Hill & R.P. Ross, (2012) Bioengineered Nisin A Derivatives with Enhanced Activity against Both Gram Positive and Gram Negative Pathogens. PloS one 7: e46884.
Field, D., T. Blake, H. Mathur, P.M. O'Connor, P.D. Cotter, R.P. Ross & C. Hill, (2018) Bioengineering Nisin to overcome the Nisin Resistance Protein. Mol Microbiol.
Field, D., N. Gaudin, F. Lyons, P.M. O'Connor, P.D. Cotter, C. Hill & R.P. Ross, (2015) A bioengineered nisin derivative to control biofilms of Staphylococcus pseudintermedius. PloS one 10: e0119684.
Field, D., P.M. O'Connor, P.D. Cotter, C. Hill & R.P. Ross, (2008) The generation of nisin variants with enhanced activity against specific gram-positive pathogens. Mol Microbiol 69: 218-230.
Field, D., L. Quigley, P.M. O'Connor, M.C. Rea, K. Daly, P.D. Cotter, C. Hill & R.P. Ross, (2010) Studies with bioengineered Nisin peptides highlight the broad-spectrum potency of Nisin V. Microb Biotechnol 3: 473-486.
Ge, X., K. Teng, J. Wang, F. Zhao, F. Wang, J. Zhang & J. Zhong, (2016) Ligand determinants of nisin for its induction activity. Journal of Dairy Science 99: 5022-5031.
Healy, B., D. Field, P.M. O'Connor, C. Hill, P.D. Cotter & R.P. Ross, (2013) Intensive mutagenesis of the nisin hinge leads to the rational design of enhanced derivatives. PloS one 8: e79563.
Kuipers, O.P., M.M. Beerthuyzen, R.J. Siezen & W.M. De Vos, (1993) Characterization of the nisin gene cluster nisABTCIPR of Lactococcus lactis. Requirement of expression of the nisA and nisI genes for development of immunity. European journal of biochemistry 216: 281-291.
Kuipers, O.P., G. Bierbaum, B. Ottenwalder, H.M. Dodd, N. Horn, J. Metzger, T. Kupke, V. Gnau, R. Bongers, P. van den Bogaard, H. Kosters, H.S. Rollema, W.M. de Vos, R.J. Siezen, G. Jung, F. Gotz, H.G. Sahl & M.J. Gasson, (1996) Protein engineering of lantibiotics. Antonie Van Leeuwenhoek 69: 161-169.
Kuipers, O.P., P.G. De Ruyter, M. Kleerebezem & W.M. De Vos, (1998) Quorum sensing-controlled gene expression in lactic acid bacteria. J Biotechnol 64: 15-21.
Li, Q., M. Montalban-Lopez & O.P. Kuipers, (2018) Increasing the Antimicrobial Activity of Nisin-Based Lantibiotics against Gram-Negative Pathogens. Appl Environ Microbiol 84.
Quigley, L., O. O'Sullivan, T.P. Beresford, R.P. Ross, G.F. Fitzgerald & P.D. Cotter, (2011) Molecular approaches to analysing the microbial composition of raw milk and raw milk cheese. Int J Food Microbiol 150: 81-94.
Rouse, S., D. Field, K.M. Daly, P.M. O'Connor, P.D. Cotter, C. Hill & R.P. Ross, (2012) Bioengineered nisin derivatives with enhanced activity in complex matrices. Microb Biotechnol 5: 501-508.
Sears, P.M., B.S. Smith, W.K. Stewart, R.N. Gonzalez, S.D. Rubino, S.A. Gusik, E.S. Kulisek, S.J. Projan & P. Blackburn, (1992) Evaluation of a nisin-based germicidal formulation on teat skin of live cows. J Dairy Sci 75: 3185-3190.
Zhou, L., A.J. van Heel, M. Montalban-Lopez & O.P. Kuipers, (2016) Potentiating the Activity of Nisin against Escherichia coli. Frontiers in Cell and Developmental Biology 4: 7.

### Example 2 - Assessing the ability of Nisin A and derivatives thereof to inhibit gram negative bacteria from the genus Thermus

### Summary

Nisin is a bacteriocin that is globally employed as a biopreservative in food systems to control gram-positive, and some gram-negative, bacteria. Here we tested the bioactivity of nisin A producing *Lactococcus lactis* NZ9700 and producers of bioengineered variants thereof against representatives of the gram-negative genus, *Thermus,* which has been associated with the pink discoloration defect in cheese. Starting with a total of 88 nisin variant-producing *Lactococcus lactis,* bioactivity against *Thermus* was assessed by agar diffusion assays, and 22 variants were found to have bioactivity greater or equal to that of the nisin A producing control. To determine to what extent this enhanced bioactivity was attributable to an increase in specific activity, minimum inhibitory concentrations (MIC's) were determined using the corresponding purified form of these 22 nisin A derivatives. From these experiments, nisin M17Q and M21F were identified as peptides with enhanced antimicrobial activity against the majority of *Thermus* target strains tested. In addition, a number of other peptide variants were found to exhibit enhanced specific activity against a subset of strains.

Identification of biopreservatives that can inhibit the growth of *Thermus* could assist in preventing the pink discolouration defect caused by the presence of *Thermus.*

### Materials and Methods

### Castenholz Media Preparation

Castenholz TYE media was chosen for selective enumeration of *Thermus* bacteria. It was prepared by mixing 5 parts of 2X concentrated Castenholz salts with 1 part of 1% TYE and 4parts of d.H₂O. Castenholz Salts (2X) contains - 0.2 g nitrilotriacetic acid, 0.12 g CaSO₄·2H₂O, 0.2 g MgSO₄.H₂O, 0.016g NaCl, 0.21g KNO₃, 1.4 g NaNO₃,0.22g Na₂HPO₄, 2.0 ml FeCl₃ solution (0.03%), and 2.0 ml Nitsch's trace elements (0.5 ml H₂SO₄, 2.2g MnSO₄, 0.5g ZnSO₄·7H₂O, 0.5g H₃BO₃, 0.016g CuSO₄·5H₂O, 0.025g Na₂MoO₄·2H₂O, 0.046g COCl₂·6H₂O distilled water [1 litre]), final volume was made up to 1 litre with d.H₂O, followed by stirring until all salts get dissolved and adjusted pH to 8.2. Media was prepared by adding 100ml of 1% TYE solute (10.0 g tryptone, 10.0 g yeast extract dissolved in 1L d.H₂O), 500 mls of Castenholz salts mixture, and 400 ml of d.H₂O. The final pH of Castenholz TYE medium was adjusted to pH 7.6. For preparation of the corresponding agar, 3% (wt/vol) bacteriological agar was added to the final solution and autoclaved at 121°C for a 15mins cycle.

### Culturing of Thermus

All *Thermus* bacterial strains used in this study were obtained from DSMZ - the German Collection of Microorganisms and Cell cultures (Braunschweig, Germany). These strains, *Thermus* thermophilus HB27 (DSMZ 7039), *Thermus scotoductus* SE-1 (DSMZ 8553), *Thermus aquaticus* YT-1 (DSMZ 625), *Thermus oshimai* SPS-17 (DSMZ 12092), *Thermus rehai* RH99-GF7504 (DSMZ 15653), *Thermus brockianus* GE-1 (DSMZ 104090), were grown using Castenholz TYE medium at 60° C in a shaking incubator for 48hrs. For plating, Castenholz media with 3% bacteriological agar was used and incubated at 55° C for 3 days.

### Culturing of Lactococcus lactis NZ9700 and derivatives thereof

The nisin A producing *L. lactic* NZ9700 and *L. lactis* NZ9700Δ*nisA* strains used to produce nisin derivatives were grown in M17 broth (Oxoid, Hants, UK) supplemented with 0.5% glucose (GM17) or GM17 agar at 30° C for overnight. All *L lactis* strains were obtained from the DPC (Dairy products centre) culture collection at Teagasc, Ireland.

### Agar diffusion assays to determine bioactivity levels

Agar diffusion assays were carried out in triplicate using nisin producing and indicator (*T. thermophilus* HB27 and *T. scotoductus* Se-1) strains. Both the producer and indicator strains were cultured as per conditions described above. One ml of overnight culture of indicators were added to 50ml of molten Castenholz agar, briefly swirled and poured. Wells were made using sterile glass pipette. To generate cell free supernatant from nisin producing strains, 10 mls of overnight cultures of the strains were centrifuged at 950g for 20 mins. The cell free supernatant was transferred to a sterile tube. 50 µl of cell free supernatant was added to wells described above. Plates were incubated at 55°C for 3 days and the zone of clearance measured.

### Nisin purification

*L. lactis* NZ9700 and variant nisin-producing strains of interest were subcultured twice in GM17 broth at 1% at 30°C before use. 2 litres of modified TY broth were inoculated with the culture at 0.5% and incubated at 30°C overnight. The culture was centrifuged at 3820g for 15 mins. The culture supernatant was applied to an Econo column containing 60 g Amberlite XAD 16 beads. The column was washed with 400 mls of 35% ethanol and inhibitory activity eluted in 400 mls of 70% 2-propanol 0.1% TFA (IPA). The 2-propanol was evaporated using a rotary evaporator (Buchi) and the sample applied to a 10g (60 ml) Strata-E C-18 Bond Elut Column (Phenomenex, Cheshire, UK) pre-equilibrated with methanol and water. The column was washed with 100 mls of 25% ethanol and the inhibitory activity was eluted in 100 mls of IPA. 10 ml aliquots were concentrated to 3 mls through removal of 2-propanol by rotary evaporation. 2 ml aliquots were applied to a Jupiter proteo (Phenomenex, Cheshire, UK) C12 reverse phase (RP)-HPLC column (4µ 90 Å, 250 x 10.0 mm, 4 µm) running at 25-45% acetonitrile, gradient where buffer A 0.1% TFA and buffer B is 100% acetonitrile 0.1% TFA. Eluent was monitored at 214 nm and fractions collected at 1 minute intervals. Nisin containing fractions were assayed for purity using MALDI-TOF mass Spectrometry and purified fractions lyophilised in a Genevac HT4 lyophiliser.

### MALDI-TOF mass spectrometry

In all cases MALDI-TOF analysis was performed with an Axima TOF² MALDI-TOF mass spectrometry (Shimadzu Biotech, Manchester, UK). A 0.5 µl aliquot of matrix solution (alpha-cyano-4-hydroxy cinnamic acid (CHCA), 10 mg/ml in 50% acetonitrile-0.1% (v/v) trifluoroacetic acid) was placed onto the target and left for 1-2 mins before being removed. The residual solution was then air dried and the sample solution was positioned onto the precoated sample spot. Matrix solution (0.5 µl) was added to the sample and allowed to air dry. The sample was subsequently analysed in positive-ion reflectron mode.

### Minimum Inhibitory Concentration assays

Minimum inhibitory concentration (MIC) determinations for gram negative *Thermus* strains were carried out in triplicate in round bottom microtitre plates (Corning). 96 well microtitre plates were pre-treated with bovine serum albumin (BSA) prior to addition of the peptides. Briefly, to each well of the microtire plates 200 µl of d.H₂O (filtered with 0.2 µm filter) containing 1% (w/w) BSA was added and incubated at 37°C for 30 mins. The wells were washed with 200 µl of IX phosphate buffered saline (PBS) for 2 times after removing 1% BSA d.H₂O. 100 µl of growth media, i.e., Castenholz, was added to each well. 0.5 mM of nisin peptides of interest was added to the first well of each row and twofold serial dilution was performed until a final concentration of 0.000975 mM was achieved. Target strains were grown overnight as per conditions mention above, 0.5 ml of overnight was subcultured in to 10 mls of fresh Castenholz medium and allowed to grow to an OD₆₀₀ of ∼0.5. 20µl of this subculture was added to 980µl of media, 150µl of this dilution added to 14.85 ml of growth media. 100µl of this dilution added to each well resulting in a final inoculum of ∼10⁵ cfu/ml in 200µl. The plates were incubated at 55° C for 3 days and the MIC was determined as the lowest concentration at which no growth was visible.

### Statistical analysis

Statistical analysis was conducted on three replicates well diffusion assays using SPSS statistics software, version 24 (IBM Crop., 2016). An ANOVA was applied to determine mean difference of clearance zone obtained by well-diffusion assays. The *Post hoc* test Dunnet >control (Keppel and Wickens, 2004) was used for pairwise comparison of treatments at level of significance P<0.05.

### Results

### Bioactivity of Nisin A and its variants against gram-negative Thermus bacterial strains

The cell free supernatant of nisin A and nisin variant producing *L. lactis* was used to determine the bioactivity of these lantibiotics against *T. thermophilus* HB27 and *T. scotoductus* SE-1 using agar diffusion assays, with the zone of clearance representing the bioactivity of the producing strain (i.e., representing a combination of the amount of the peptide produced and the specific activity of that peptide). Notably, the nisin A producer was active against the *Thermus* strains despite their gram negative cell wall. We compared the bioactivity of producers of bioengineered nisin A variants with changes in the N-terminal domain, C-terminal domain and hinge region with the producer of wild type nisin A. Starting with screening of 88 nisin A derivative-producing strains, we observed that 22 producers showed bioactivity that was greater or equal to that of the nisin A producer (Table 1 and Table 2). Among the variants tested were those with altered N-terminal domains. The N-terminal domain of nisin is important for binding with the pyrophosphate moiety of lipid II to mediate pore formation in the cell membrane. Lysine (K) at position 12, which serves as a small flexible region between ring B and C, and is one of among the 5 positively charged residues (K12, K22, K34, H27, H3) of nisin A that contributes to its cationic nature and its attraction to the cell envelope. Variant-containing supernatants in which either hydrophilic (Threonine T, Serine S, Tyrosine Y, Glutamine Q) or hydrophobic (Leucine L, Methionine M, valine V) amino acid residues replaced K12 were studied, and it was established that K12T and K12V exhibited significantly greater bioactivity compared to that of the wild type nisin A producer against *T. thermophilus* HB27 (Table: 1) and *T. scotoductus* SE-1 (Table: 2).

Research by Weidemann et al. (2001) showed that the introduction of a positive charge at position 17 to replace Methionine (M) reduced the antimicrobial activity of Nisin Z M17K to 50% (Wiedemann et al., 2001). Here the activity of Nisin A M17Q, where a hydrophobic residue was replaced by hydrophilic neutral residue glutamine (Q), was assessed and no enhancement in bioactivity against *Thermus* was apparent.

The hinge region of nisin, i.e., Asparagine 20, Methionine 21, Lysine 22, is a flexible stretch linking the N-terminal and C-terminal domains, its structural role is not clearly understood, NMR studies revealed that the hinge region of nisin Z is important for the translocation of the C-terminal across the membrane. Here it was established that producers of M21A, M21V (also known as nisin V), VGA and PAQ (Table: 4) exhibit statistically significantly enhanced bioactivity against *T. thermophilus* HB27. In contrast, it is the producers of nisin A M21Q, AAK, VGA that exhibited significantly better activity than wild type producer against *T. scotoductus* SE-1 (Table: 5).

The C-terminal domain of nisin is responsible for active pore formation. Like Lysine (K) at position 12, Histidine (H) at position 27 is one of 5 positively charged residues in nisin. Producers of a peptide in which the Imidazole H27 was converted to the polar neutral Glutamine (Q) displayed statistically significantly better bioactivity against *T. thermophilus* HB27 and higher bioactivity than wild type for *T. scotoductus* SE-1. Furthermore, with respect to the C-terminus, producers of a peptide in which the polar negatively charged Aspartic acid (D) was introduced in place of Serine 29 showed significantly enhanced activity against both *T. thermophilus* HB27 and *T. scotoductus* SE-1 (Table 4 and 5). This result is consistent with studies performed by Field, et al (2012) where they established that negatively charged substitution at position 29, i.e., S29E, S29D variants of nisin A, showed enhanced specific activity against gram-negative bacteria (Field et al., 2012).

Although, other variants such as K12L, K12S K12M, N20S, M21F, M21Q, M21Y, PGA have better antibacterial activity than wild type producer against *T*. *thermophilus* HB27, and K12S, K12M, N20P, N20S, M21F, M21Y, M21V, VGV have higher antimicrobial activity than wild type producer against *T*. *scotoductus* SE-1, these enhancements are not statistically significant.

### Specific activity of Nisin A and its variants against gram-negative Thermus

The bioactivity of cell-free supernatant can be influenced by altered diffusion rates, solubility, production or specific activity. To determine to what extent, if any, the bioactivity noted above is due to increased specific activity, nisin A (control) and the derivatives were purified and their molecular weights confirmed by MALDI TOF mass spectrometry (Table: 6) (Figure: 4). These peptides were used to perform minimum inhibitory concentration assays against six *Thermus* strains, i.e., *T. thermophilus* HB27, *T. scotoductus* SE-1, *T. rehai, T. oshimai, T. aquaticus* and *T. brockianus.*

MICs obtained are the mean of triplicate experiments tested against the target strains (Table: 7) (Figure: 5). N-terminal domain nisin A variants tested were K12T, K12V, K12S, K12Y, K12M, K12L, M17Q. The MIC for nisin A peptide against *T. thermophilus* HB27, *T. oshimai,* and *T rehai* was 26.1 µg/L, 52.3 µg/L for *T. brockianus, T. aquaticus* and 209.6 µg/L for *T. scotoductus* SE-1. K12V peptide displayed 2 fold better activity than nisin A against *T*. *thermophilus* HB27, *T. scotoductus* SE-1, *T rehai,* (MIC's 12.9 µg/L , 103.9 µg/L, 122.9 µg/L respectively). M17Q peptide showed better activity against all the strains tested, exhibiting 2 fold better activity against *T. brockianus* and *T. aquaticus* (6.5 µg/L, 26.2 µg/L, respectively), 4 fold against *T. scotoductus* SE-1 (52.28 µg/L) and *T. rehai* (6.53 µg/L) and an 8 fold better activity against *T. thermophilus* HB27 (3.2 µg/L) and *T. oshimai* (3.2 µg/L). K12S showed 2 fold better activity against *T. scotoductus* SE-1 (103.5 µg/L) only. K12Y displayed 2 fold and 4 fold better activity against *T. aquaticus* (26.4 µg/L) and *T. rehai* (6.6 µg/L) respectively, while K12L was 2 fold (26 µg/L) more active than nisin A against *T. aquaticus.*

Hinge region nisin A variants N20P, N20S, M21V, M21A, M21F, M21Y, M21F, AAK, PAQ, VGA, and PGA were compared with nisin A against target strains. N20S exhibited enhanced activity (2 to 8 fold) against 5 of the targeted strains, the exception being *T. brockianus.* The introduction of polar neutral residues (Y, Q) at position M21 resulted in enhanced activity against *T*. *oshimai, T. rehai, T. aquaticus* (MICs for M21Y were 13.2 µg/L, 13.2 µg/L, 6.6 µg/L and those for M21Q were 6.5 µg/L, 13 µg/L, 26.1 µg/L, respectively), while M21Y was two-fold more active against *T. thermophilus* HB27 (13.2 µg/L). M21F showed better antimicrobial activity against all of the target strains with a 2 to 8 fold difference compared to wild type.

The C-terminal domain nisin A variants tested were H27Q and S29D. Of these H27Q showed an 8 fold enhanced activity against *T. thermophilus* HB27, *T. oshimai, T. rehai* (3.2 µg/L) and 14 fold enhanced activity against *T. aquaticus* (3.26 µg/L).

### Discussion

Nisin is a ribosomally synthesized bacteriocin that is approved for use as a food biopreservative by many agencies. It exhibits a broad spectrum of bactericidal activity against many gram-positive bacteria, including food pathogens and its mode of action involves the creation of pores in the cell membrane and/or the inhibition of peptidoglycan synthesis by preventing transpeptidation. In this regard, the importance of the lipid II molecule has been well studied. The activity of nisin against gram-negative bacteria is diminished due to the presence of the lipopolysaccharide (LPS) in outer membrane (OM), which acts as a barrier and restricts the entry of the nisin peptide. Nisin, when used in combination with chelating agents like EDTA (Stevens et al., 1991), at high salt concentrations environment (Kuwano et al., 2005), food grade oils, or together with other hurdle technology systems, can be used to control gram-negatives. While Qian et al. (2018) highlighted the merits of combining full/truncated nisin with pore forming peptides to improve activity against gram-negative pathogens (Li et al., 2018), to date, only a few studies have identified bioengineered nisin variants that in isolation exhibit enhanced activity against gram negatives (Field et al., 2012; Yuan et al., 2004). Here we identified bioengineered nisin A variants with enhanced bactericidal activity against gram-negative *Thermus* strains. More specifically, we identified bioengineered nisin A variants of the N-terminal domain (K12V, M17Q, K12S, K12L, and K12Y), hinge region (N20S, M21F, M21Y, M21Q) and the C-terminal domain (H27Q) that exhibited enhanced specific activity against *Thermus* strains. Moreover, M17Q and M21F showed enhanced antimicrobial activity against all *Thermus* strains tested, and represent the first example of nisin peptides with aromatic residue that exhibit enhanced activity. Field et al (2012) suggested that the increased activity of nisin derivatives against gram-negative bacteria was due to an increased ability of nisin to traverse the OM (Field et al., 2012). This could be the case with *Thermus* also. It is also worth noting that *Thermus* contains exceptionally large porins, which might contribute to the sensitivity of the strains to nisin, and bioengineered derivatives thereof (Nikaido, 1996). Other factors such as the lack of lipopolysaccharide (LPS), instead contains polar glycolipids (GL) and phosphoglycolipids (PGL) with C₁₆,C₁₈,C₁₄ saturated and unsaturated fatty acids, is a feature of some strains of genus *Thermus.* This might also have a significant role in enhancing nisin activity, as LPS is thought to contribute to the nisin resistance of gram-negative bacteria (Nikaido, 1996). Further research is needed to identify the mechanism of action of nisin peptides against *Thermus,* and why certain derivatives possess greater potency than others.

Pink discoloration in cheese is a significant problem to cheese industries internationally, with the associated pink band under the surface or all over the cheese leading to product rejection by consumers. Semi-hard cheeses like Emmental, Cheddar (both coloured and non-coloured), Romano, Gouda etc. are susceptible to this discoloration. Many reasons for this discoloration have been postulated by researchers and include an interaction among metabolites released by microbes present in cheese, high nitrates and tyrosine, photochemical oxidation and the presence of pigments produced by cheese microbes. For more details on pink discoloration, the reader is directed towards the review by Daly et al on pink discolouration in commercial cheese (Daly et al., 2012). A recent metagenomic study within our group identified high levels of carotenoid producing *Thermus thermophilus* in Swiss-type pink defective cheese. This species was not regarded as being cheese-associated, (Quigley et al., 2016). Ultimately, the current investigation to determine the ability of nisin and bioengineered variants thereof to inhibit the growth of *Thermus* bacteria could be a useful discovery for cheese industries. Nisin had been using as a biopreservative in dairy products like cheese, and is known to inhibit pathogens like *L. monocytogenes* and *Clostridium difficile* in cheese matrices. A similar application of bioengineered nisin A variants to inhibit the growth of *Thermus* in cheese could possibly help in preventing pink defect caused by the presence of this bacterium, especially as strains that have been bioengineered through self-cloning to produce a bacteriocin with single amino acid change are not regulated as genetically modified microorganisms (GMMs) (Field et al., 2018). It is noted that further research will need to be done to check the activity of these nisin peptides in cheeses against *Thermus.*

### Tables

**Table 4: Bioactivity of Nisin A producing Lactococcus lactis and its variants against Thermus thermophilus HB27. Results are expressed as zone diameter in mm. * denotes activity greater then wild type nisin A. values in bold reached statistical significance compared to a nisin control (level of significance P<0.05).**

| **Position and Nature of Residue** | ***Lactococcus lactis* Strain** | ***Thermus thermophilus* HB27** | |
|---|---|---|---|
| | | **Zone Diameter (mm)** | **% difference (p value)** |
| | Nisin A wild type | 6 ±1. 7 | 100 |
| **N-terminal domain** | K 12 T | 8.93±1.3 | **148* (.001)** |
| | K 12 S | 7.50±2.2 | 125 |
| Hydrophilic: Neutral | K 12 Y | 5.67±0.9 | 94 |
| | M 17 Q | 7.97±1.1 | 132 |
| Hydrophobic | K 12 L | 7.20±1.3 | 120 |
| | K 12 M | 7.10±1.5 | 118 |
| | K 12 V | 8.70±0.8 | **145* (0.004)** |
| **Hinge Region** | N 20 S | 7.17±0.9 | 119 |
| Hydrophilic : Neutral | M 21 Q | 7.40±1.3 | 123 |
| | M 21 Y | 6.93±0.8 | 115 |
| | N 20 P | 5.83±1.8 | 97 |
| Hydrophobic | M 21 F | 7.52±0.9 | 125 |
| | M 21 A | 9.50±0.9 | **158* (0.001)** |
| | M 21 V | 8.27±0.9 | **137* (0.022)** |
| 2 Hydrophobic, 1 Hydrophilic | VGA | 10.17±1.4 | **169* (0.0002)** |
| | PAQ | 8.30±1.4 | **138* (0.02)** |
| | PGA | 6.83±0.8 | 113 |
| **C-Terminal domain** | H 27 Q | 8.17±0.7 | **136* (0.031)** |
| Hydrophilic: neutral and -ve charged | S 29 D | 10.17±1.5 | **169* (0.0001)** |

**Table 5: Bioactivity Nisin A producing Lactococcus lactis and its variants against Thermus scotuductus Se-1. Results are expressed as zone diameter in mm. * denotes activity greater then wild type nisin A. values in bold reached statistical significance compared to a nisin control (level of significance P<0.05)**

| **Position and Nature of Residue** | ***Lactococcus lactis* Strain** | ***Thermus scotoductus* SE-1** | |
|---|---|---|---|
| | | **Zone Diameter (mm)** | **% of wild type (p value)** |
| | Nisin A wild type | 6.5 ± 0.5 | 100 |
| **N-terminal domain** | K 12 T | 9.1 ± 1 | **140* (0.001)** |
| | K 12 Y | 4.80 ± 0.2 | 74 |
| Hydrophilic: Neutral | K 12 S | 8 ± 0.01 | 123 |
| | K 12 V | 8.10 ± 0.9 | **125* (0.04)** |
| Hydrophobic | K 12 M | 7.65 ± 1 | 118 |
| **Hinge Region** | N 20 S | 7.25 ± 0.9 | 112 |
| Hydrophilic : Neutral | M 21 Q | 8.05 ± 1 | **124* (0.05)** |
| | M 21 Y | 7.50 ± 0.5 | 115 |
| | N 20 P | 7.50 ± 0.6 | 115 |
| | M 21 F | 6.90 ± 0.8 | 106 |
| Hydrophobic | M 21 V | 7.75 ± 0.9 | 119 |
| | M 21 M | 7.65 ± 0.7 | 118 |
| 2 Hydrophobic, 1 Hydrophilic | AAK | 8.60 ± 0.9 | **132* (0.004)** |
| | VGA | 8.25 ± 0.3 | **127* (0.022)** |
| | VGV | 7.45 ± 1 | 115 |
| **C-Terminal domain** | H 27 Q | 7.50 ± 0.5 | 115 |
| Hydrophilic: neutral and -ve charged | S 29 D | 8.15 ± 0.6 | **125* (0.033)** |

**Table 6: MALDI TOF mass spectrometry analysis of Nisin A and its variants used in this study for MIC's assays.**

| | **Molecular mass** | |
|---|---|---|
| **Strain** | **Predicted** | **Obtained** |
| **Nisin A Wild-type** | 3354.07 | 3353.55 |
| **AAK** | 3250.92 | 3251.59 |
| **K12T** | 3326.99 | 3326.63 |
| **K12V** | 3325.03 | 3324.33 |
| **M21V** | 3322.02 | 3321.52 |
| **K12S** | 3312.97 | 3312.58 |
| **N20P** | 3337.08 | 3336.70 |
| **S29D** | 3382.09 | 3381.61 |
| **M21A** | 3293.96 | 3291.17 |
| **N20S** | 3327.05 | 3325.52 |
| **M21F** | 3370.05 | 3368.29 |
| **K12Y** | 3389.07 | 3388.32 |
| **PAQ** | 3276.93 | 3276.93 |
| **M21Y** | 3385.66 | 3384.70 |
| **M21Q** | 3350.61 | 3350.64 |
| **VGA** | 3207.863 | 3205.30 |
| **K12L** | 3339.05 | 3337.98 |
| **H27Q** | 3342.67 | 3344.14 |
| **PGA** | 3205 | 3203.79 |
| **K12M** | 3357.07 | 3357.32 |
| **M17Q** | 3351.07 | 3349.18 |

**Table 7: MIC's of pure Nisin A and its variant peptides against Thermus bacterial strains. Results are expressed as a mean of triplicate assays. * represents 2 fold to 6 fold lower MIC's compared to wild type. WT: wild type.**

| | **MIC µg/L (µM)** | | | | | |
|---|---|---|---|---|---|---|
| **Nisin Peptides** | ***T. thermophilus HB27*** | ***T. scotoductus* SE-1** | ***T. oshimai*** | ***T. rehai*** | ***T. brockianus*** | ***T. aquaticus*** |
| Nisin A(WT) | 26.16 (0.0078) | 209.63 (0.0625) | 26.16 (0.0078) | 26.16 (0.0078) | 52.32 (0.0156) | 52.32 (0.0156) |
| K12S | 103.53 (0.0312) | **103.53* (0.0312)** | 51.68 (0.0156) | 51.68 (0.0156) | 103.53 (0.0312) | 103.53 (0.0312) |
| K12T | 51.90 (0.0156) | 207.94 (0.0625) | 103.97 (0.0312) | 51.90 (0.0156) | 207.94 (0.0625) | 103.97 (0.0312) |
| K12V | **12.97* (0.0039)** | **103.91* (0.0312)** | 51.87 (0.0156) | **12.97* (0.0039)** | 51.87 (0.0156) | 51.87 (0.0156) |
| K12L | 52.09 (0.0156) | 208.69 (0.0625) | 26.04 (0.0078) | 26.04 (0.0078) | 104.35 (0.0312) | **26.04*** (0.0078) |
| K12Y | 52.87 (0.0156) | 211.82 (0.625) | 52.87 (0.0156) | **6.61* (0.00195)** | 105.91 (0.0312) | **26.43*** (0.0078) |
| K12M | 52.37 (0.0156) | 419.63 (0.125) | 52.37 (0.0156) | 26.19 (0.0078) | 209.82 (0.0625) | 52.37 (0.0156) |
| M17Q | **3.27* (0.000975)** | **52.28* (0.0156)** | **3.27* (0.000975)** | **6.53* (0.00195)** | **26.14*** (0.0078) | **26.14*** (0.0078) |
| N20S | **12.98* (0.0039)** | **103.97* (0.3125)** | **3.24* (0.000975)** | **6.49* (0.00195)** | 51.90 (0.0156) | **25.95*** (0.0078) |
| N20P | 52.06 (0.0156) | 417.14 (0.125) | 208.57 (0.0625) | 208.57 (0.0625) | 208.57 (0.0625) | 52.06 (0.0156) |
| M21Q | 13.07 (0.0039) | 209.41 (0.0625) | **6.53* (0.00195)** | **13.07* (0.0039)** | 52.27 (0.0156) | **26.13*** (0.0078) |
| M21A | 102.94 (0.0312) | 102.94 (0.03120) | 25.69 (0.0078) | 102.94 **(0.0312)** | 205.87 (0.0625) | 25.69 (0.0078) |
| M21F | **13.14* (0.0039)** | **105.31* (0.0312)** | **6.57* (0.00195)** | **3.29* (0.000975)** | **26.29*** (0.0078) | **13.14* (0.0039)** |
| M21Y | **13.20* (0.0039)** | 211.60 (0.0625) | **13.20* (0.0039)** | **13.20* (0.0039)** | 105.80 (0.0312) | **6.60* (0.00195)** |
| M21V | 25.91(0.0078) | 207.63 (0.0625) | 25.91 (0.0078) | 25.91(0.0078) | 103.81 (0.0312) | 103.81 (0.0312) |
| AAK | 101.59 (0.0312) | 203.18 (0.0625) | 50.71 (0.0156) | 50.71 (0.0156) | 50.71 (0.0156) | 203.18 (0.0625) |
| PAQ | 25.56 (0.0078) | 204.81 (0.0625) | **12.78* (0.0039)** | 51.12 (0.0156) | 102.40 (0.0312) | 51.12 (0.0156) |
| VGA | 100.25 (0.0312) | 400.98 (0.125) | 50.04 (0.0156) | 400.98 (0.125) | 400.98 (0.125) | 50.04 (0.0156) |
| PGA | 50.00 (0.0156) | 400.63 (0.125) | 25.00 (0.0078) | 50.00 (0.0156) | 200.31 (0.0625) | 100.16 (0.0312) |
| H27Q | **3.26* (0.000975)** | 208.92 (0.0625) | **3.26* (0.000975)** | **3.26* (0.000975)** | 52.15 (0.0156) | **3.26* (0.000975)** |
| S29D | 105.69 (0.0312) | 211.38 (0.0625) | 26.38 (0.0078) | 26.38 (0.0078) | 105.69 (0.0312) | 105.69 (0.0312) |

### References

Daly, D. F. M., McSweeney, P. L. H., and Sheehan, J. J. (2012). Pink discolouration defect in commercial cheese: A review. Dairy Sci. Technol. 92, 439-453. doi:10.1007/s13594-012-0079-0.
Davies, E. A., and Bevis, H. E. (2010). The Use of the Bacteriocin, Nisin, as a Preservative in Ricotta-type Cheeses to Control the Food-borne Pathogen Listeria monocytogenes TP 2010-1e. 343-346.
Field, D., Begley, M., O'Connor, P. M., Daly, K. M., Hugenholtz, F., Cotter, P. D., et al. (2012). Bioengineered Nisin A Derivatives with Enhanced Activity against Both Gram Positive and Gram Negative Pathogens. PLoS One 7. doi:10.1371/journal.pone.0046884.
Field, D., Blake, T., Mathur, H., O' Connor, P. M., Cotter, P. D., Paul Ross, R., et al. (2019). Bioengineering nisin to overcome the nisin resistance protein. Mol. Microbiol. 111, 717-731. doi:10.1111/mmi.14183.
Field, D., Cotter, P. D., Ross, R. P., and Hill, C. (2015). Bioengineering of the model lantibiotic nisin. Bioengineered 6, 187-192. doi:10.1080/21655979.2015.1049781.
Field, D., Ross, R. P., and Hill, C. (2018). Developing bacteriocins of lactic acid bacteria into next generation biopreservatives. Curr. Opin. Food Sci. 20, 1-6. doi:10.1016/j.cofs.2018.02.004.
Keppel, G., and Wickens, T. D. (2004). Chapter 6: Simultaneous Comparisons and the Control of Type I Errors*.*
Kuwano, K., Tanaka, N., Shimizu, T., Nagatoshi, K., Nou, S., and Sonomoto, K. (2005). Dual antibacterial mechanisms of nisin Z against Gram-positive and Gram-negative bacteria. Int. J. Antimicrob. Agents 26, 396-402. doi:10.1016/j.ijantimicag.2005.08.010.
Li, Q., Montalban-lopez, M., and Kuipers, P. (2018). Increasing the Antimicrobial Activity of Nisin-Based Lantibiotics against Gram-Negative pathogens. 84, 1-15.
Molloy, E. M., Field, D., O'Connor, P. M., Cotter, P. D., Hill, C., and Ross, R. P. (2013). Saturation Mutagenesis of Lysine 12 Leads to the Identification of Derivatives of Nisin A with Enhanced Antimicrobial Activity. PLoS One 8. doi:10.1371/journal.pone.0058530.
Nikaido, H. (1996). Outer membrane. In: Neidhardt F C, Curtiss III R, Ingraham J L, Lin E C C, Low K B Jr, Magasanik B, Reznikoff W S, Riley M, Schaechter M, Umbarger H E, editors. Escherichia coli and Salmonella: cellular and molecular biology. 2nd ed. Washington, D.C: Amer. Microbiol. Mol. Biol. Rev. 67, 29-47. doi:10.1128/MMBR.67.4.593.
Quigley, L., O 'Sullivan, D. J., Daly, D., O 'Sullivan, O., Burdikova, Z., Vana, R., et al. (2016). Thermus and the Pink Discoloration Defect in Cheese. mSystems 1(3), e00023-16. doi:10.1128/mSystems.00023-16.
Stevens, K. A., Sheldon, B. W., Klapes, N. A., and Klaenhammer, T. R. (1991). Nisin treatment for inactivation of Salmonella species and other gram-negative bacteria. Appl. Environ. Microbiol. 57, 3613-3615.
Wiedemann, I., Breukink, E., Van Kraaij, C., Kuipers, O. P., Bierbaum, G., De Kruijff, B., et al. (2001). Specific binding of nisin to the peptidoglycan precursor lipid II combines pore formation and inhibition of cell wall biosynthesis for potent antibiotic activity. J. Biol. Chem. 276, 1772-1779. doi:10.1074/jbc.M006770200.
Yuan, J., Zhang, Z. Z., Chen, X. Z., Yang, W., and Huan, L. D. (2004). Site-directed mutagenesis of the hinge region of nisinZ and properties of nisinZ mutants. Appl. Microbiol. Biotechnol. 64, 806-815. doi:10.1007/s00253-004-1599-1.

All references herein are incorporated by reference.

### Wild-type nisin sequences

Nisin A
   ITSISLCTPGCKTGALMGCNMKTATCHCSIHVSK (3354 Da) (SEQ ID NO: 1).
Nisin Z
   ITSISLCTPGCKTGALMGCNMKTATCNCSIHVSK (3331 Da) (SEQ ID NO: 21).
Nisin F
   ITSISLCTPGCKTGALMGCNMKTATCNCSVHVSK (3315 Da) (SEQ ID NO: 22).
Nisin Q
   ITSISLCTPGCKTGVLMGCNLKTATCNCSVHVSK (3327 Da) (SEQ ID NO: 23).
Nisin H
   FTSISMCTPGCKTGALMTCNYKTATCHCSIKVSK (3453 Da) (SEQ ID NO: 24).
Nisin J
   ITSKSLCTPGCKTGALQTCFAKTATCHCSGHVHTK (3458 Da) (SEQ ID NO: 25).
Nisin U
   ITSKSLCTPGCKTGILMTCPLKTATCGCHFG (3029 Da) (SEQ ID NO: 26).
Nisin U2
   VTSKSLCTPGCKTGILMTCPLKTATCGCHFG (3015 Da) (SEQ ID NO: 27).
Nisin P
   VTSKSLCTPGCKTGILMTCAIKTATCGCHFG (2989 Da) (SEQ ID NO: 28).
Nisin 01,2,3
   YKSKSACTPGCPTGILMTCPLKTATCGCHITGK (3546 Da) (SEQ ID NO: 29).
Nisin O4
   TSQHSFCTPNCLTGFLCP-PKTQLTCTCKLKGQ (3259 Da) (SEQ ID NO: 30).

## Claims

1. A variant nisin peptide, wherein the variant nisin peptide is a modified variant of wild-type nisin, wherein the modification comprises or consists of an amino acid residue modification selected from:
i) M17Q or M17A;
ii) T2L; and
iii) one set of modified hinge residues selected from a) N20H and M21T, b) N20V, M21G, and K22A, c) N20V, M21G, and K22V or d) N20V, M21G, and K22T; or
iv) combinations of modifications i), ii) and iii).

2. A variant nisin peptide, wherein the variant nisin peptide is a modified variant of wild-type nisin, wherein the modification comprises or consists of an amino acid residue modification selected from:
i) M17Q;
ii) K12T or K12V
iii) N20S
iv) one of M21F, M21A, M21V, M21Y, or M21Q;
v) H27Q; and
vi) one set of modified hinge residues selected from a) N20V, M21G, and K22A, or b) N20P, M21A, and H27Q; or
vii) combinations of modifications i) to vi).

3. The variant nisin peptide according to claim 1 or claim 2, wherein the modification further comprises a modification selected from I4V, K12A, K12T, K12S, K12P, A15G, M17S, N20P, N20K, N20E, M21A, M21V, M21K, K22A, K22T, K22S, K22G, H27Q, S29G, S29A, S29D, S29E, S29R, S29P, and H31K, or combinations thereof; and/or
wherein the modification further comprises a modification to S29 and/or 130; and/or
wherein the modification further comprises a modification where NMK hinge residues are replaced by one of PAL, PMQ, PTL, PMC, HLT, GVK. AAA, SAA, NAI, AAK, SLS, SMT, NAT, QVQ, PMA, PVN, SIN, ASS, ANP, SVA, PIT, PAQ, PGA, and PAK.

4. A nucleic acid encoding the variant nisin peptide according to any preceding claim.

5. A composition comprising a variant nisin according to any one of claims 1-3, or comprising the nucleic acid according to claim 4, optionally wherein the composition is a pharmaceutical composition comprising a pharmaceutically acceptable carrier; and/or
wherein the composition is suitable for topical administration to skin, oral administration, injection, or intramammary infusion; and/or
wherein the composition is in the form of an ointment, cream/emulsion, paste, lotion, gel, or teat seal.

6. A container comprising the composition according to claim 5 for treatment or prevention of mastitis, optionally wherein the container is a syringe, pipette, or dropper.

7. A wipe impregnated with one or more forms of variant nisin peptides according to any one of claims 1-3, or the composition according to claim 5.

8. A kit comprising:
- a nisin variant peptide according to any one of claims 1-3, or the composition according to claims 5; and
- an applicator, such as a syringe, pipette, dropper, or wipe.

9. A variant nisin according to any one of claims 1-3, or the composition according to claim 5, for use as a medicament.

10. A variant nisin peptide according to any one of claims 1-3, or the composition according to claim 5, for use in the treatment or prevention of a bacterial infection in a subject, or for use in reducing bacterial colonisation of a subject.

11. A method of treatment or prevention of a bacterial infection in a subject, wherein the method comprises the administration of the variant nisin peptide according to any one of claims 1-3, or the composition according to claim 5, to the subject.

12. The use of the Nisin variant peptide according to any one of claims 1-3, or the composition according to claim 5, as a preservative, such as for food, or for reducing or inhibiting the growth of bacterial species, such as *Thermus spp,.* in one or more of food, beverage, healthcare product or cosmetic product, ingredients thereof, packaging thereof and preparation equipment thereof.

13. A method of preserving food, beverage, healthcare product or cosmetic product. against spoilage from bacterial species, such as *Thermus spp.,* the method comprising the addition of the Nisin variant peptide according to any one of claims 1-3, or the composition according to claim 5, to one or more of the food, beverage, healthcare product or cosmetic product, ingredients thereof, packaging thereof and preparation equipment thereof.

14. A cell that is capable of expressing one or more variant nisin peptides according to any one of claims 1-3, or comprises the nucleic acid according to claim 4.

15. A method of producing the variant nisin peptide according to any one of claims 1-12, the method comprising expressing variant nisin peptide in a cell culture that has been modified to produce the variant nisin peptide.


SEQUENCE LISTING

<110>  University College Cork-National University of Ireland
       TEAGASC, the Agricultural and Food Development Authority

<120>  Variant Nisin Peptides

<130>  JDM98355P.EPA

<140>  EP19208735.1
<141>  2019-11-12

<160>  30

<170>  PatentIn version 3.5

<210>  1
<211>  34
<212>  PRT
<213>  Lactococcus lactis

<400>  1

Ile Thr Ser Ile Ser Leu Cys Thr Pro Gly Cys Lys Thr Gly Ala Leu
1               5                   10                  15

Met Gly Cys Asn Met Lys Thr Ala Thr Cys His Cys Ser Ile His Val
            20                  25                  30

Ser Lys

<210>  2
<211>  34
<212>  PRT
<213>  Artificial Sequence

<220>
<223>  Engineered peptide

<400>  2

Ile Thr Ser Ile Ser Leu Cys Thr Pro Gly Cys Lys Thr Gly Ala Leu
1               5                   10                  15

Gln Gly Cys Asn Met Lys Thr Ala Thr Cys His Cys Ser Ile His Val
            20                  25                  30

Ser Lys

<210>  3
<211>  34
<212>  PRT
<213>  Artificial Sequence

<220>

<223>  Engineered peptide

<400>  3

Ile Leu Ser Ile Ser Leu Cys Thr Pro Gly Cys Lys Thr Gly Ala Leu
1               5                   10                  15

Met Gly Cys Asn Met Lys Thr Ala Thr Cys His Cys Ser Ile His Val
            20                  25                  30

Ser Lys

<210>  4
<211>  34
<212>  PRT
<213>  Artificial Sequence

<220>
<223>  Engineered peptide

<400>  4

Ile Thr Ser Ile Ser Leu Cys Thr Pro Gly Cys Lys Thr Gly Ala Leu
1               5                   10                  15

Met Gly Cys His Thr Lys Thr Ala Thr Cys His Cys Ser Ile His Val
            20                  25                  30

Ser Lys

<210>  5
<211>  34
<212>  PRT
<213>  Artificial Sequence

<220>
<223>  Engineered peptide

<400>  5

Ile Thr Ser Ile Ser Leu Cys Thr Pro Gly Cys Lys Thr Gly Ala Leu
1               5                   10                  15

Met Gly Cys Val Gly Ala Thr Ala Thr Cys His Cys Ser Ile His Val
            20                  25                  30

Ser Lys

<210>  6
<211>  34
<212>  PRT

<213>  Artificial Sequence

<220>
<223>  Engineered peptide

<400>  6

Ile Thr Ser Ile Ser Leu Cys Thr Pro Gly Cys Lys Thr Gly Ala Leu
1               5                   10                  15

Met Gly Cys Val Gly Val Thr Ala Thr Cys His Cys Ser Ile His Val
            20                  25                  30

Ser Lys

<210>  7
<211>  34
<212>  PRT
<213>  Artificial Sequence

<220>
<223>  Engineered peptide

<400>  7

Ile Thr Ser Ile Ser Leu Cys Thr Pro Gly Cys Lys Thr Gly Ala Leu
1               5                   10                  15

Met Gly Cys Val Gly Thr Thr Ala Thr Cys His Cys Ser Ile His Val
            20                  25                  30

Ser Lys

<210>  8
<211>  34
<212>  PRT
<213>  Artificial Sequence

<220>
<223>  Engineered peptide

<400>  8

Ile Leu Ser Ile Ser Leu Cys Thr Pro Gly Cys Lys Thr Gly Ala Leu
1               5                   10                  15

Gln Gly Cys Asn Met Lys Thr Ala Thr Cys His Cys Ser Ile His Val
            20                  25                  30

Ser Lys

<210>  9
<211>  34
<212>  PRT
<213>  Artificial Sequence

<220>
<223>  Engineered peptide

<400>  9

Ile Thr Ser Ile Ser Leu Cys Thr Pro Gly Cys Lys Thr Gly Ala Leu
1               5                   10                  15

Gln Gly Cys His Thr Lys Thr Ala Thr Cys His Cys Ser Ile His Val
            20                  25                  30

Ser Lys

<210>  10
<211>  34
<212>  PRT
<213>  Artificial Sequence

<220>
<223>  Engineered peptide

<400>  10

Ile Thr Ser Ile Ser Leu Cys Thr Pro Gly Cys Lys Thr Gly Ala Leu
1               5                   10                  15

Gln Gly Cys Val Gly Ala Thr Ala Thr Cys His Cys Ser Ile His Val
            20                  25                  30

Ser Lys

<210>  11
<211>  34
<212>  PRT
<213>  Artificial Sequence

<220>
<223>  Engineered peptide

<400>  11

Ile Thr Ser Ile Ser Leu Cys Thr Pro Gly Cys Lys Thr Gly Ala Leu
1               5                   10                  15

Gln Gly Cys Val Gly Val Thr Ala Thr Cys His Cys Ser Ile His Val
            20                  25                  30

Ser Lys

<210>  12
<211>  34
<212>  PRT
<213>  Artificial Sequence

<220>
<223>  Engineered peptide

<400>  12

Ile Thr Ser Ile Ser Leu Cys Thr Pro Gly Cys Lys Thr Gly Ala Leu
1               5                   10                  15

Gln Gly Cys Val Gly Thr Thr Ala Thr Cys His Cys Ser Ile His Val
            20                  25                  30

Ser Lys

<210>  13
<211>  34
<212>  PRT
<213>  Artificial Sequence

<220>
<223>  Engineered peptide

<400>  13

Ile Leu Ser Ile Ser Leu Cys Thr Pro Gly Cys Lys Thr Gly Ala Leu
1               5                   10                  15

Met Gly Cys His Thr Lys Thr Ala Thr Cys His Cys Ser Ile His Val
            20                  25                  30

Ser Lys

<210>  14
<211>  34
<212>  PRT
<213>  Artificial Sequence

<220>
<223>  Engineered peptide

<400>  14

Ile Leu Ser Ile Ser Leu Cys Thr Pro Gly Cys Lys Thr Gly Ala Leu
1               5                   10                  15

Met Gly Cys Val Gly Ala Thr Ala Thr Cys His Cys Ser Ile His Val
            20                  25                  30

Ser Lys

<210> 15
<211> 34
<212> PRT
<213> Artificial Sequence

<220>
<223> Engineered peptide

<400> 15

Ile Leu Ser Ile Ser Leu Cys Thr Pro Gly Cys Lys Thr Gly Ala Leu
1               5                   10                  15

Met Gly Cys Val Gly Val Thr Ala Thr Cys His Cys Ser Ile His Val
            20                  25                  30

Ser Lys

<210> 16
<211> 34
<212> PRT
<213> Artificial Sequence

<220>
<223> Engineered peptide

<400> 16

Ile Leu Ser Ile Ser Leu Cys Thr Pro Gly Cys Lys Thr Gly Ala Leu
1               5                   10                  15

Met Gly Cys Val Gly Thr Thr Ala Thr Cys His Cys Ser Ile His Val
            20                  25                  30

Ser Lys

<210> 17
<211> 34
<212> PRT
<213> Artificial Sequence

<220>
<223> Engineered peptide

<400> 17

Ile Leu Ser Ile Ser Leu Cys Thr Pro Gly Cys Lys Thr Gly Ala Leu
1               5                   10                  15

Gln Gly Cys His Thr Lys Thr Ala Thr Cys His Cys Ser Ile His Val
            20                  25                  30

Ser Lys

<210>  18
<211>  34
<212>  PRT
<213>  Artificial Sequence

<220>
<223>  Engineered peptide

<400>  18

Ile Leu Ser Ile Ser Leu Cys Thr Pro Gly Cys Lys Thr Gly Ala Leu
1               5                   10                  15

Gln Gly Cys Val Gly Ala Thr Ala Thr Cys His Cys Ser Ile His Val
            20                  25                  30

Ser Lys

<210>  19
<211>  34
<212>  PRT
<213>  Artificial Sequence

<220>
<223>  Engineered peptide

<400>  19

Ile Leu Ser Ile Ser Leu Cys Thr Pro Gly Cys Lys Thr Gly Ala Leu
1               5                   10                  15

Gln Gly Cys Val Gly Val Thr Ala Thr Cys His Cys Ser Ile His Val
            20                  25                  30

Ser Lys

<210>  20
<211>  34
<212>  PRT
<213>  Artificial Sequence

<220>
<223>  Engineered peptide

<400>  20

Ile Leu Ser Ile Ser Leu Cys Thr Pro Gly Cys Lys Thr Gly Ala Leu
1               5                   10                  15

Gln Gly Cys Val Gly Thr Thr Ala Thr Cys His Cys Ser Ile His Val
            20                  25                  30

Ser Lys

<210>  21
<211>  34
<212>  PRT
<213>  Lactobacillus lactis

<400>  21

Ile Thr Ser Ile Ser Leu Cys Thr Pro Gly Cys Lys Thr Gly Ala Leu
1               5                   10                  15

Met Gly Cys Asn Met Lys Thr Ala Thr Cys Asn Cys Ser Ile His Val
            20                  25                  30

Ser Lys

<210>  22
<211>  34
<212>  PRT
<213>  Lactobacillus lactis

<400>  22

Ile Thr Ser Ile Ser Leu Cys Thr Pro Gly Cys Lys Thr Gly Ala Leu
1               5                   10                  15

Met Gly Cys Asn Met Lys Thr Ala Thr Cys Asn Cys Ser Val His Val
            20                  25                  30

Ser Lys

<210>  23
<211>  34
<212>  PRT
<213>  Lactobacillus lactis

<400>  23

Ile Thr Ser Ile Ser Leu Cys Thr Pro Gly Cys Lys Thr Gly Val Leu
1               5                   10                  15

Met Gly Cys Asn Leu Lys Thr Ala Thr Cys Asn Cys Ser Val His Val
            20                  25                  30

Ser Lys

<210> 24
<211> 34
<212> PRT
<213> Lactobacillus lactis

<400> 24

Phe Thr Ser Ile Ser Met Cys Thr Pro Gly Cys Lys Thr Gly Ala Leu
1               5                   10                  15

Met Thr Cys Asn Tyr Lys Thr Ala Thr Cys His Cys Ser Ile Lys Val
            20                  25                  30

Ser Lys

<210> 25
<211> 35
<212> PRT
<213> Lactobacillus lactis

<400> 25

Ile Thr Ser Lys Ser Leu Cys Thr Pro Gly Cys Lys Thr Gly Ala Leu
1               5                   10                  15

Gln Thr Cys Phe Ala Lys Thr Ala Thr Cys His Cys Ser Gly His Val
            20                  25                  30

His Thr Lys
        35

<210> 26
<211> 31
<212> PRT
<213> Lactobacillus lactis

<400> 26

Ile Thr Ser Lys Ser Leu Cys Thr Pro Gly Cys Lys Thr Gly Ile Leu
1               5                   10                  15

Met Thr Cys Pro Leu Lys Thr Ala Thr Cys Gly Cys His Phe Gly
            20                  25                  30

<210> 27
<211> 31
<212> PRT
<213> Lactobacillus lactis

<400>  27

Val Thr Ser Lys Ser Leu Cys Thr Pro Gly Cys Lys Thr Gly Ile Leu
1               5                   10                  15

Met Thr Cys Pro Leu Lys Thr Ala Thr Cys Gly Cys His Phe Gly
            20                  25                  30

<210>  28
<211>  31
<212>  PRT
<213>  Lactobacillus lactis

<400>  28

Val Thr Ser Lys Ser Leu Cys Thr Pro Gly Cys Lys Thr Gly Ile Leu
1               5                   10                  15

Met Thr Cys Ala Ile Lys Thr Ala Thr Cys Gly Cys His Phe Gly
            20                  25                  30

<210>  29
<211>  33
<212>  PRT
<213>  Lactobacillus lactis

<400>  29

Tyr Lys Ser Lys Ser Ala Cys Thr Pro Gly Cys Pro Thr Gly Ile Leu
1               5                   10                  15

Met Thr Cys Pro Leu Lys Thr Ala Thr Cys Gly Cys His Ile Thr Gly
            20                  25                  30

Lys

<210>  30
<211>  32
<212>  PRT
<213>  Lactobacillus lactis

<400>  30

Thr Ser Gln His Ser Phe Cys Thr Pro Asn Cys Leu Thr Gly Phe Leu
1               5                   10                  15

Cys Pro Pro Lys Thr Gln Leu Thr Cys Thr Cys Lys Leu Lys Gly Gln
            20                  25                  30